(19) Europäisches Patentamt / European Patent Office / Office européen des brevets

(11) **EP 3 087 516 B1**

(12) **EUROPEAN PATENT SPECIFICATION**

(45) Date of publication and mention
of the grant of the patent:
**07.11.2018 Bulletin 2018/45**

(51) Int Cl.:
*G06F 19/00* (2018.01)   *G01N 21/27* (2006.01)
*G01N 21/359* (2014.01)   *G01N 35/00* (2006.01)
*G01N 21/65* (2006.01)

(21) Application number: **14830387.8**

(22) Date of filing: **23.12.2014**

(86) International application number:
**PCT/EP2014/079152**

(87) International publication number:
**WO 2015/097217 (02.07.2015 Gazette 2015/26)**

(54) **METHOD AND SYSTEM FOR PREPARING SYNTHETIC MULTICOMPONENT BIOTECHNOLOGICAL AND CHEMICAL PROCESS SAMPLES**

ENTWICKLUNG MULTIVARIATER KALIBRIERUNGEN UND/ODER ÜBERWACHUNGSSYSTEME

DÉVELOPPEMENT D'ÉTALONNAGES MULTIVARIABLES ET/OU SYSTÈMES DE SURVEILLANCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.12.2013 EP 13199699**

(43) Date of publication of application:
**02.11.2016 Bulletin 2016/44**

(73) Proprietor: **F. Hoffmann-La Roche AG
4070 Basel (CH)**

(72) Inventors:
• **CARDOSO-MENEZES, Jose
P-1070-237 Lisbon (PT)**
• **BUZIOL, Stefan
82515 Wolfratshausen (DE)**
• **FELIZARDO, Pedro
P-2685-227 Portela LRS (PT)**

(74) Representative: **Vossius & Partner
Patentanwälte Rechtsanwälte mbB
Siebertstrasse 3
81675 München (DE)**

(56) References cited:
• DE JUAN A. ET AL: "Chemometrics applied to unravel multicomponent processes and mixtures: Revisiting latest trends in multivariate resolution", ANALYTICA CHIMICA ACTA, vol. 500, no. 1-2, 1 January 2003 (2003-01-01), pages 195-210, XP055005359, ISSN: 0003-2670 cited in the application
• BLANCO M. ET AL: "Analysis of pharmaceuticals by NIR spectroscopy without a reference method", TRAC, TRENDS IN ANALYTICAL CHEMISTRY, ELSEVIER, AMSTERDAM, NL, vol. 29, no. 10, 1 November 2010 (2010-11-01), pages 1127-1136, XP027409049, ISSN: 0165-9936 [retrieved on 2010-08-18]
• AZZOUZ T. ET AL: "Application of multivariate curve resolution alternating least squares (MCR-ALS) to the quantitative analysis of pharmaceutical and agricultural samples", TALANTA, ELSEVIER, AMSTERDAM, NL, vol. 74, no. 5, 18 January 2008 (2008-01-18), pages 1201-1210, XP022426331, ISSN: 0039-9140, DOI: 10.1016/J.TALANTA.2007.08.024
• TAULER R. ET AL: "Multivariate Curve Resolution Applied to Spectral Data from Multiple Runs of an Industrial Process", ANALYTICAL CHEMISTRY.A, AMERICAN CHEMICAL SOCIETY. COLUMBUS, US, vol. 65, no. 15, 15 August 1993 (1993-08-15), pages 2040-2047, XP002386198,

EP 3 087 516 B1

• RILEY M. R. ET AL: "Rapid Calibration of Near-Infrared Spectroscopic Measurements of Mammalian Cell Cultivations", BIOTECHNOLOGY PROGRESS, vol. 15, no. 6, 29 October 1999 (1999-10-29), pages 1133-1141, XP055122742, ISSN: 8756-7938, DOI: 10.1021/bp990117v
• BEEBE K. R. ET AL: "An Introduction to Multivariate Calibration and Analysis", ANALYTICAL CHEMISTRY, AMERICAN CHEMICAL SOCIETY, US, vol. 59, no. 17, 1 September 1987 (1987-09-01), pages 1007A-1017A, XP009102134, ISSN: 0003-2700, DOI: 10.1021/AC00144A001
• FEUDALE R. N. ET AL: "Transfer of multivariate calibration models: a review", CHEMOMETRICS AND INTELLIGENT LABORATORY SYSTEMS, ELSEVIER SCIENCE PUBLISHERS B.V. AMSTERDAM, NL, vol. 64, no. 2, 28 November 2002 (2002-11-28), pages 181-192, XP004388757, ISSN: 0169-7439, DOI: 10.1016/S0169-7439(02)00085-0

• HOPKE P. K.: "The evolution of chemometrics", ANALYTICA CHIMICA ACTA, vol. 500, no. 1-2, 19 December 2003 (2003-12-19), pages 365-377, XP055119684, ISSN: 0003-2670, DOI: 10.1016/S0003-2670(03)00944-9
• ESCANDAR G. M. ET AL: "Second- and third-order multivariate calibration: data, algorithms and applications", TRAC, TRENDS IN ANALYTICAL CHEMISTRY, ELSEVIER, AMSTERDAM, NL, vol. 26, no. 7, 8 August 2007 (2007-08-08), pages 752-765, XP022188800, ISSN: 0165-9936, DOI: 10.1016/J.TRAC.2007.04.006

**Description**

Technical Field

The present invention relates to methods for preparing synthetic multicomponent samples of powders, liquids and liquids with suspended components that can mimic dynamic processes, particularly multicomponent mixtures, crystallizations, distillations, chemical reactions and fermentations, to be used in the development of multivariate calibrations and multivariate supervision systems of such processes. In particular, the present invention relates to a method for developing multivariate calibrations and/or supervision systems and more particularly to a computer program and a system for running such a method.

Background Art

Monitoring, supervision and control of industrial processes is often difficult and time consuming since these systems have complex multicomponent matrices that require laboratory chemical and physical analysis by reference methods. Furthermore, these determinations often have a low acquisition frequency compared to process dynamics, are highly time and resources consuming offline techniques and do not allow process monitoring and control.

Recently the use of spectroscopic data allowed the quality control (chemical and physical) to be readily available (on-line) at the process (in-situ) instead of being determined in the laboratory (offline). The use of these on-line and multi-parametric techniques has proved to be capable of determining process trajectories and multi-parameter analysis (chemical and physical) in almost real-time. This can be a great advantage over reference analytical laboratory methods, since spectroscopic sensors can enable the implementation of process control strategies.

Spectroscopic sensors perform multidimensional measurements that through the use of multivariate data analysis, also called chemometrics, can be related with specific chemical or physical parameters, or even dynamic process profiles. These relations are established by multivariate calibrations that can be used either to monitor or supervise processes and can be a part of advanced process control.

Although spectroscopic sensors present an advantage over reference analytical laboratory methods, they require the development of robust calibrations that can establish the relation between the spectral data and a specific parameter, which is called a multivariate calibration (linear or nonlinear). Moreover, spectral data can be used without any specific parameter to establish multivariate dynamic process profiles.

Multivariate calibrations are usually developed either by using in-line process spectral data (X) and in-process samples that are withdrawn from the process and then analyzed by offline analytical methods (y) or by using samples prepared by orthogonal design of experiments. Disregarding the used method for multivariate calibrations, the dataset to build them should comprise data from samples that allow the development of calibrations that meet the following requisites as good as possible:

Spectral Selectivity - The calibration should be selective for each calibrated variable, predicting it with accuracy in any stage of a process and even predict outside its range. Variability - The calibration data should include as much variability as possible to ensure model validity over a wide range of conditions. Matrix effects should also be included. Matrix effects can be considered as the combined effect of all components (known and unknown) within the sample on its spectra, e.g., considering different media solutions for a calibration or different solvents in mixture. Uniformity - The calibration samples should have a distribution as even as possible across the expected calibration range. This will increase the quality of the calibration by avoiding extreme samples (sometimes seen by the model as outlier samples). Non-correlativity - The existence of correlations between parameters can give rise to lack of selectivity.

Even though the use of process samples is usually the most common option for the development of multivariate calibrations for spectroscopic sensors, there are several drawbacks behind it. For example, samples from these processes are comparably complex matrixes of known and sometimes unknown compounds (multicomponent). Or processes dynamics and sampling frequency do not match, for instance, high number of samples with no process variation and a low number of samples when the process presents a high dynamic change. Or high number of samples are required to have a significant process variability (several batches or operation modes are needed). Or high amount of offline analytical measurements are needed. Or high correlation coefficients between some of the parameters are present in the calibration samples (such as reaction profile composition). Or processes can take several days (for instance fermentations).

To overcome these drawbacks or at least some of them and meet the necessary requests of good calibrations as good as possible, orthogonal designs of experiments directly imported from statistic literature, have been proposed to produce non-process derived samples. These designs guarantee that all the experiments are independent, meeting the above mentioned requisites but commonly yield comparably poor calibration results for spectroscopy sensors. Also, these designs usually only have 3 to 5 levels of variation, which often is not enough for the calibration of these sensors. Furthermore, these orthogonal methods only can be used for known and measurable variations, which is not the case when considering process samples where a considerable amount of unknown and non-measurable variations are present.

In the article "Analysis of pharmaceuticals by NIR spectroscopy without a reference method" by Marcelo Blanco and Anna Peguero, Trends in Analytical Chemistry, Volume 29, Issue 10, November 2010, Pages 1127-1136, refers to constructing calibration sets for quantifying the active pharmaceutical ingredient (API) and excipients in pharmaceutical tablets. The operating procedure involves using an appropriate experimental design to prepare a set of laboratory samples and recording a series of NIR spectra during the pharmaceutical-production process. Process spectra are calculated by difference between the NIR spectra for production tablets and those for laboratory samples containing API and excipients at their nominal concentrations.

The article "Rapid calibration of near-infrared spectroscopic measurements of mammalian cell cultivations" by M. R. Riley et al., Biotechnology Progress, vol. 15, no. 6, pages 1133-1141, relate to an approach for generating NIR spectroscopic calibrations wherein a small number of experimentally collected spectra serve as inputs to a computational procedure that yields a large number of simulated spectra, each containing both analyte-specific and analyte-independent information.

The article "An Introduction to Multivariate Calibration and Analysis" by K. R. Beebe et al., Anal. Chem., 1987, 59 (17), pp 1007A-1017A, refers to a general introduction to multivariate calibration and analysis using multiple linear regression, principal component regression as well as partial least square.

The article "Transfer of multivariate calibration models: a review" by R. N. Feudale et al., Chemometrics and Intelligent Laboratory Systems, Volume 64, Issue 2, 28 November 2002, Pages 181-192, refers to an overview of the different methods used for calibration transfer and a critical assessment of their validity and applicability.

The article "The evolution of chemometrics" by P.K. Hopke, Analytica Chimica Acta Volume 500, Issues 1-2, 19 December 2003, Pages 365-377, the development of chemometrics as a subfield of chemistry and particularly analytical chemistry is presented.

The article "Second- and third-order multivariate calibration: data, algorithms and applications" by G. M. Escandar et al. refers to a review of second- and third-order multivariate calibration, based on the growing literature in the field, the variety of data being produced by modern instruments, and the proliferation of algorithms capable of dealing with higher-order data.

Therefore, there is a need for a method for preparing synthetic multicomponent samples that can mimic dynamic processes to be used in the development of comparably accurate and robust multivariate calibrations and of multivariate supervision systems of such processes. Also there is a need for a method which allows making these calibrations and supervision systems comparably accurate and robust.

Disclosure of the Invention

An object of the invention is to provide a method for preparing at least one synthetic multicomponent biotechnological and/or chemical process sample and to provide a synthetic sample layout generation and sample handling system for preparing a set of synthetic samples mimicking a dynamic process or a specific process step or a variation thereof. Another object of the invention is to provide a computer program product comprising one or more computer readable media having computer executable instructions for performing the steps of the aforementioned method.

The object of the invention is solved with the features of the independent claims. Dependent claims refer to preferred embodiments.

As described in more detail below, the present invention makes use, among others, of Multivariate Curve Resolution - Alternating Least Squares (MCR-ALS), which is a chemometric method or algorithm that is commonly used for the resolution of multicomponent responses in unknown unresolved mixtures as, e.g. described in [3]. The MCR-ALS method or algorithm aims for the description of multicomponent systems by using a bilinear model that relates the system composition with multivariate responses, such as spectroscopic measurements, electrochemical signals, etc. (see, e.g., [3]).

In order to better understand and explain the method according to the invention, a brief description of the MCR-ALS algorithm is given below. A more detailed description of such algorithm can be found in [1] or in [3].

MCR-ALS, a bilinear modelling method, is generally used to decompose an experimental data matrix D into the product of two orthogonal matrices: a matrix C related with the "true" pure responses profiles associated with the variation contribution of each component of the original data matrix which is usually related with the changes in chemical composition or the amount of solution in the mixture, and a matrix $S^T$ related with the observed data variation such as, for instance, instrumental measures or spectroscopic changes. The simulation resolves Equation 1 below iteratively by the Alternating Least Squares (ALS) algorithm, calculating the pure responses profiles C and the observed variations $S^T$ optimally fitting the experimental data matrix D, while minimizing the matrix of residuals E that cannot be described by the model or algorithm.

$$D = CS^T + E \qquad \text{(Equation 1)}$$

The optimization procedure requires the user to establish a number of components to be used in the model and an initial estimate of C or $S^T$, which can be achieved by using chemometrics methods such as Principal Component Analysis, Evolving Factor Analysis (EFA) or SIMPLISMA, or other suitable methods.

Since the MCR-ALS method is an ambiguous method, incorporating iterative procedures for constraints implementation can be a good option to reduce its ambiguity, i.e., to decrease the number of feasible solutions that fit equally well the experimental data. Commonly used constraints for matrices C and $S^T$ can include non-negativity, unimodality, closure, trilinearity, selectivity and/or other constraints that are known. The inclusion of such constraints helps finding meaningful physical or chemical solutions for the problems to be solved.

The MCR-ALS algorithm optimization performance is evaluated by the percentage of lack of fit (Equation 2), defined as the difference between the original data D and the product of matrices C and $S^T$, the percentage of variance explained by the model (Equation 3) and the standard deviation of the residuals (Equation 4). The following equations show how to calculate the performance of the MCR-ALS algorithm or model:

$$\text{Lack of fit} = 100 \sqrt{\frac{\sum_{i,j} e_{ij}^2}{\sum_{i,j} d_{ij}^2}} \qquad \text{(Equation 2)}$$

$$R^2 = \frac{\sum_{i,j} d_{ij}^2 - \sum_{i,j} e_{ij}^2}{\sum_{i,j} d_{ij}^2} \qquad \text{(Equation 3)}$$

$$\sigma = \sqrt{\frac{\sum_{i,j} e_{ij}^2}{n_{rows}\, n_{columns}}} \qquad \text{(Equation 4)}$$

where, $e_{ij}$ is the residual obtained from the difference between the element at position ij of matrix D and the estimate from the MCR-ALS model or algorithm, $d_{ij}$ is the valued of the element at position ij of matrix D and $n_{rows}$ and $n_{columns}$ designate the number of rows and columns of matrix D.

In particular, the invention relates to a method for preparing at least one synthetic multicomponent biotechnological and/or chemical process sample for developing multivariate calibrations for monitoring systems and/or multivariate supervisory systems of increased robustness, wherein at least one synthetic sample mimicking a dynamic process or a specific process step or a variation thereof is prepared, the method comprising the steps of: preparing historical process data of the dynamic process or the specific process step or the variation thereof to be mimicked and for which the at least one synthetic sample is to be prepared; creating a data matrix D of the historical process data; determining a plurality of main solutions of the data matrix D; determining which main solutions of the data matrix D are necessary to mimic the dynamic process or the specific process step or the variation thereof within a predetermined variance; determining the analyte composition of the necessary main solutions and the respective relative amount of the necessary main solutions with respect to the data matrix D; creating a matrix S comprising the determined analyte composition of the necessary main solutions; creating a matrix C comprising the relative amount of the necessary main solutions with respect to the data matrix D; determining whether the matrix product C x $S^T$ corresponds to the data matrix D within a predetermined tolerance; determining the relative amount of the necessary main solutions comprised in the matrix C for at least two instants of time; performing a regression between the relative amount of the necessary main solutions comprised in the matrix C and a respective time variable of the historical process data using the determined relative amount of the necessary main solutions for the at least two instants of time; estimating the relative amount of the necessary main solutions for at least one other instant of time between the at least two instants of time based on the regression; and creating an augmented time dependent matrix $C_{aug}$ comprising C and the estimated necessary main solutions for the at least one other instant of time, wherein the aforementioned steps are carried out on a computational unit; and creating at least one sample by assembling the necessary main solutions according to the determined analyte composition.

Preferably, creating the at least one sample further comprises the step of mixing the assembled necessary main solutions according to their determined respective relative amount.

Preferably, preparing historical process data comprises arranging data to a specific format, for instance sorting by time or according to process evolution.

Preferably, creating a least one sample comprises producing, assembling, mixing and/or preparing the at least one sample.

The historical process data of the dynamic process or specific process step or a variation thereof can be obtained, e.g., by measuring parameters in the dynamic process or specific process step, by averaging several runs of dynamic processes or specific process steps, by gathering such data in the literature or the like.

For example, the obtained historical process data can comprise analytically determined composition values of the data matrix of the dynamic process or the specific process step or a variation thereof. Such data allows for efficiently and in some cases sufficiently perform the method.

The obtained historical process data of the dynamic process or the specific process step or a variation thereof can also comprise physical and/or process information.

For example, the physical and/or process information can be pH values, temperature values or the like. Such data also allows for efficiently and in some cases sufficiently perform the method.

The obtained historical process data of the dynamic process or the specific process step or a variation thereof can also be organized according to a process time. Organizing the historical process data in such manner allows - in the case of a batch or fed-batch process step or dynamic changes in a continuous process - for obtaining time profiles for the specific process step.

The obtained historical process data of the dynamic process or the specific process step or a variation thereof further can also comprise data of at least one further specific process step corresponding to an experimental run of the specific process step under similar or different process conditions. Like this, a data matrix can be formed which can be used as matrix D in the MCR-ALS algorithm described above.

Using factor decomposition algorithm allows for determining how many main solutions are necessary to mimic the dynamic process or the specific process step or the variation thereof within a predetermined variance.

The predetermined variance can be the optimum accumulated captured variance and can be set according to good modelling practices known by the skilled person, e.g. a chemometrics specialist. For example, it can be set that the predetermined variance, i.e. the accumulated captured variance, is between 95 to 99%. In other words, it can be determined how many main solutions of the data matrix D are necessary to mimic the dynamic process or the specific process step or the variation thereof within a predetermined variance, in this example between 95 to 99%.

Preferably, an evolving factor analysis algorithm is used for the decomposition of the data matrix, i.e. the determination of how many main solutions are necessary to mimic the dynamic process or the specific process step or the variation thereof within a predetermined variance. Such determination allows for efficiently choosing the number of relevant main solutions and determines where they appear or disappear during the dynamic process, the specific process step or a variation thereof, in the case of naturally ordered datasets such as most batch, semi-batch and dynamic changes in continuous processes.

Preferably, the optimum accumulated variance is determined within decomposing the data matrix using a factor decomposition method, such as PCA, evolving factor analysis or other suitable method and the optimum accumulated captured variance is evaluated whether to be within a predefined range or not. Determining the optimum accumulated variance or accumulated captured variance in such a range allows for obtaining the optimum number of multivariate main solutions that have to be used in the method according to the invention. If the optimum accumulated captured variance is within the predefined range, the optimum of the number of multivariate main solutions can be obtained. If the used factor decomposition method is not able to capture such accumulated variance, then the data matrix or matrix D can be split into smaller data matrices as described below and these new datasets can be used in the further steps of the method according to the invention. Thereby, the predefined range of the optimum accumulated variance can be preferably more than 94% and more preferably from 95% to 99%. Determining the optimum accumulated captured variance in said range allows for particularly efficiently obtaining the optimum number of multivariate main solutions that have to be used in the method according to the invention.

Preferably, an appearance time of the number of main solutions and/or a disappearance time of the number of main solutions is determined within the decomposition of the data matrix using evolving decomposition methods. Such appearance or disappearance time allows for efficiently performing the method.

The initial estimation of analyte composition of the necessary main solutions and the initial estimation of the respective relative amount of the necessary main solutions with respect to the data matrix D can be determined by evolving factor analysis and/or determination of the purest sample/variable. An example for an evolving factor analysis can be found in [2]; an example for the determination of the purest sample/variable can be found in [4].

In particular, the plurality of main solutions of the data matrix D can be determined using an appropriate chemometric method. The chemometric method can be a factor decomposition method, in particular principal component analysis (PCA), singular value decomposition (SVD) or evolving factor analysis, and/or a parallel factor analysis (PARAFAC), multivariate curve resolution-alternating least squares (MCR-ALS), evolving factor analysis.

The predetermined tolerance can be a value according to good modelling practices known by the skilled person. For example, such a tolerance can be 95%. In other words, it is determined whether the matrix product $C \times S^T$ corresponds to the data matrix D to or above 95%, i.e. the deviation of the corresponding matrix entries can be 5% or less.

If the matrix product $C \times S^T$ does not correspond to the data matrix D within the predetermined tolerance the following steps are preferably repeated: determining which main solutions of the data matrix D are necessary to mimic the dynamic process or the specific process step or the variation thereof within the predetermined variance; determining the analyte composition of the necessary main solutions and the relative amount of each necessary main solution; creating the matrix S; creating the matrix C; determining whether the matrix product $C \times S^T$ corresponds to the data matrix D within the predetermined tolerance. For example, if it is determined that the predetermined tolerance, e.g. 95%, is not reached, the aforementioned steps according to said embodiment can be repeated until the predetermined tolerance is met. With said tolerance the itineration performance can be evaluated.

The relative amount of the necessary main solutions for creating the data matrix C and/or the determined analyte composition of the necessary main solutions for creating the matrix S may be selected based on logical constraints and/or physical constraints and/or chemical constraints. For example, a constraint can be that an analyte concentration cannot be negative, i.e. the non-negative constraint. Another example is that for determining the relative amount of each main solution the non-negative constraint should be met and the sum of all fractions should be equal to 1, i.e. the closure constraint.

Preferably, the method comprises the steps of assembling the necessary main solutions comprised in $C_{aug}$ and mixing the necessary main solutions comprised in $C_{aug}$ according to the determined respective relative amount.

Preferably, estimating the relative amount of the necessary main solution comprises computing an unknown value by establishing regression between at least two adjacent values.

Preferably, the relative amount of each main solution for the determined number of main solutions at a specific time within deconvoluting the data matrix, e.g. by a MCR-ALS algorithm with imposed constraints, can be obtained by regression between the relative amount of each main solution predicted, e.g. by the multivariate curve resolution alternating least squares algorithm, and the time variable from the original process data. The time variable can be relative or absolute. The regressions can be a linear, polynomial or other regression.

Thereby, mixtures with the relative amount of the multicomponent of each main solution for the determined number of main solutions at the specific time preferably are obtained. The mixtures can be rows of C. The specific time in this context can be any given time between the beginning and the end of the dynamic process, the specific process step or a variation thereof, comprised in D or part of such process comprised in $D_i$. (An example of such procedure is given in Example 1 and 2 below.) Such obtaining of the mixtures allows for obtaining more samples than the MCR-ALS algorithm by itself, as such C can be augmented into $C_{aug}$ where the amount of each main solution for a given time obtained from the MCR-ALS algorithm and from the regression procedure are combined. This allows for increasing the number of samples to be run in the experimental implementation procedure.

Preferably, each of the number of main solutions can be added for a known process time after assembling each main solution for the determined number of main solutions according to the MCR-ALS results loadings matrix. In particular, after all main solutions are assembled with the characteristics determined by the MCR-ALS algorithm the dynamic process or the specific process step or a variation thereof or part of it can be mimicked by the addition of the amount of each main solution as determined by $C_{aug}$ for a known process time. The $C_{aug}$ matrix can be a p x n matrix where p is the number of samples to be created, here accounted as the sum of MCR-ALS algorithm samples (equal to the rows of matrix D) and the number of samples generated by the regression of C against time, and n is the number of main solutions that, e.g., was established by PCA or Evolving Factor Analysis. Thus, each row of $C_{aug}$ can indicate the relative amount of each main solution (presented in its columns) that has to be mixed to recreate the original samples in matrix D or the augmented samples obtained from the regression of C against time. By mixing the amount of main solution for each row, one can mimic the complete process and recreate all the samples necessary to build a library for multivariate calibration and supervision systems development. For that, a multivariable analytical device such as, e.g., comprised in the synthetic sample preparing system described below, mostly a spectroscopic sensor or any other multichannel instrument, may have to take measurements for each of the recreated samples. The obtained instrumental measurements (x) and the sample composition (y) or time (t) can be used to establish multivariate calibrations using, for example, Projection into Latent Structures regression (PLS) or for establishing supervision systems, for example, Multivariable Statistical Process Control (MSPC). Although these are comparably important methods used for multivariate calibrations and supervision systems, the present invention is not limited to these specific methods. Moreover, the present invention is also not limited to linear multivariate calibration methods. In fact, further chemometric methods can benefit from the creation of such samples in this manner, such as other linear and nonlinear regression methods, and classification methods, such as, PCA, Projection into Latent Structures Discriminant Analysis (PLS-DA), Soft Independent Modelling of Class Analogy (SIMCA) or other. Based on the method according to the present invention, discriminant methods can be developed. For example, process phases may be established based on prior knowledge and using created samples to develop discriminant and/or classification methods. New samples (e.g. from new process runs) could then be classified using these methods.

The data matrix D may be partitioned into a plurality of data matrices $D_i$ and wherein each matrix $D_i$ is individually processed in each step following the creating of the data matrix D, preferably if it is determined that only a limited number of main solutions can be used to mimic the dynamic process or the specific process step or the variation thereof at that the limited number of main solution present a variance below the predetermined variance. For example, the number of main solutions is limited to 2 or 3 by a binary or ternary mixture device with automated control, the data matrix D is partitioned into a plurality of data matrices $D_i$ and wherein each matrix Di is individually processed in each step following the creating of the data matrix D.

Preferably, individually processed in this context comprises that each of the data matrices $D_i$ are treated separately from the other sub-matrices $D_i$.

For another example, if the number of main solutions is set manually according to experimental limitation, e.g. to 3 or 4, the data matrix D is partitioned into a plurality of data matrices $D_i$ and wherein each matrix $D_i$ is individually processed in each step following the creating of the data matrix D.

Another example is that it is determined that the model used for decomposing the data matrix D is not capable to capture a predetermined variance, i.e. if it is determined that the number of main solutions for describing the data matrix D which are configured to mimic the dynamic process or the specific process step or the variation thereof are below the predetermined variance. In this example, the matrix D can be partitioned into a plurality of data matrices $D_i$ and wherein each matrix $D_i$ is individually processed in each step following the creating of the data matrix D.

Therefore, the data matrix can be split in a plurality of smaller data matrices and each one of the plurality of smaller data matrices is individually processed in each step following the decomposition of the data matrix using a factor decomposition method, such as PCA, evolving factor analysis or other suitable method. As explained above, in some complex situations, the number of main solutions may not be practical to be implemented in an experimental setup either automatically, such as a binary or ternary mixture device with automated control where the number of main mixtures is limited to two or three, respectively, or manually where the number of main solutions cannot be too high. In these cases the number of main solutions can be set manually according to experimental limitation, which may oblige to divide the complete process or process step in smaller parts as needed, i.e., dividing the data matrix or matrix D into smaller matrices $D_i$. Thereby, the data matrix can be split in the plurality of smaller data matrices if the optimum accumulated variance is determined not to be within the predefined range.

The historical data may comprise at least one of the following: analytically determined composition values of the dynamic process or the specific process step or the variation thereof, physical information, process information, data of at least one further process variation corresponding to additional experimental and/or simulated runs of the process under similar or different process conditions and/or wherein the historical data is organized in the matrix D according to the process time.

Preferably, organized in this context comprises the sorting of the historical data.

The step of determining the necessary main solutions and/or determining the analyte composition of the necessary main solutions and/or determining the relative amount of the necessary main solutions with respect to the data matrix D may be performed using an appropriate chemometric method, in particular at least one of the following chemometric methods: a factor decomposition method, in particular principal component analysis (PCA), singular value decomposition (SVD) or evolving factor analysis, and/or a parallel factor analysis (PARAFAC), multivariate curve resolution-alternating least squares (MCR-ALS), evolving factor analysis.

The method may further comprise the step of preprocessing the matrix D, preferably filtering, more preferably using at least one of: a Savitzky-Golay filter, a Kernel smoother, a smoothing spline, a moving average, or a weighted moving average. Preferably, preprocessing further comprises the application of a chemometric preprocessing algorithm.

In particular, the data matrix or matrix D can be preprocessed by using any suitable algorithm, e.g., for smoothing, mean centering, auto scaling, or the like, all of them known and used in the field of chemometrics. In some cases, a smoothing of data from one point to the other might be recommended, as an example, like the Savitsky-Golay filter algorithm can be used as a smoothing pre-processing method. This can be important when dealing with dynamic processes or specific process step or a variation thereof, since the process does not pass abruptly from one stage to the other. In some cases, the use of such preprocessing allows minimizing outlier points that are not aligned with the general trend of the dataset. This step can be of importance in order to have good results in the following steps. Although the data can be pre-processed, sometimes this is not necessary, especially if the data is only comprised of analytical composition in the same units and no outliers are enclosed in the dataset.

The method may further comprise the step of preparing at least one second sample being configured to break co-linearity between parameters or analytes comprised in the data matrix D using a co-linearity breaking method.

The co-linearity breaking method preferably comprises at least one of the following: creating of an orthogonal design of experiments, random spiking of the specific compounds in the at least one sample, programmed spiking, random mixing of samples or any combination thereof.

Preferably, random spiking comprises adding a random amount of a known chemical compound in order to break correlations. The amount of the compound to be introduced can be determined computationally.

According to another embodiment, the method may further comprise the step of creating a third sample by grouping the sample with the at least one second sample.

The invention also relates to a synthetic sample generation system and sample handling system for preparing at least one synthetic sample mimicking a dynamic process or a specific process step or a variation thereof. The system is adapted to carry out the method according to any one of the preceding embodiments and comprises: a computational unit; and a mixing unit; wherein the computational unit comprises: means for preparing historical process data of the dynamic process or the specific process step or the variation thereof to be mimicked and for which the at least one synthetic sample is to be prepared; means for creating a data matrix $D$ of the historical process data; means for determining a plurality of main solutions of the data matrix $D$; means for determining which main solutions of the data matrix $D$ are necessary to mimic the dynamic process or the specific process step or the variation thereof within a predetermined variance; means for determining the analyte composition of the necessary main solutions and the respective relative amount of the necessary main solutions with respect to the data matrix $D$; and wherein the mixing unit is configured to create the at least one sample by assembling the necessary main solutions according to the determined analyte composition, wherein the computational unit further comprises means for creating a matrix $S$ comprising the determined analyte composition of the necessary main solutions; means for creating a matrix $C$ comprising the relative amount of the necessary main solutions with respect to the data matrix $D$; means for determining whether the matrix product $C \times S^T$ corresponds to the data matrix $D$ within a predetermined tolerance, wherein the means for determining the respective relative amount of the necessary main solutions with respect to the data matrix $D$ are configured to determine the relative amount of the necessary main solutions comprised in the matrix $C$ for at least two instants of time, wherein the computational unit further comprises means for performing a regression between the relative amount of the necessary main solutions comprised in the matrix $C$ and a respective time variable of the historical process data using the determined relative amount of the necessary main solutions for the at least two instants of time; means for estimating the relative amount of the necessary main solutions for at least one other instant of time between the at least two instants of time based on the regression; and means for creating an augmented time dependent matrix $C_{aug}$ comprising $C$ and the estimated necessary main solutions for the at least one other instant of time.

Preferably, the mixing unit is configured to create the at least one sample by assembling the necessary main solutions according to the determined analyte composition and by mixing the assembled necessary main solutions according to their determined respective relative amount.

The system may further comprise a measurement device having at least one sensor. Thereby, parameters which are useful for many dynamic processes can efficiently be measured.

The at least one sensor of the measurement device may be configured to measure at least one parameter in-situ in the dynamic process, the specific process step or the variation thereof and/or the at least one sensor may comprise at least one of the following: a pH probe, a temperature probe, a spectroscopic sensor, or a multichannel instrument.

The system may further comprise at least one control device comprising at least one temperature control structure and/or at least one pH control structure. With such a control device the conditions can be defined and adjusted. Thereby, the at least one control device preferably is adapted to recreate the conditions of the dynamic process or the specific process step or the variation thereof.

The at least one control device is preferably configured to recreate respective conditions of the dynamic process or the specific process step or the variation thereof.

The at least one control device may also be configured to break the co-linearity between parameters or analytes comprised in the data matrix $D$ using a co-linearity breaking method.

Preferably, the at least one control device is configured to break the co-linearity using at least one of the following co-linearity breaking methods: creating of an orthogonal design of experiments, random spiking of the specific compounds in the at least one sample, programmed spiking, random mixing of samples or any combination thereof.

The system may further comprise an analytical device configured to analyze at least one of the main solutions and/or the at least one sample.

Preferably, the mixing unit is configured to assemble the necessary main solutions comprised in $S$ and to mix the necessary main solutions comprised in $C_{aug}$ according to the determined respective relative amount.

The invention also relates to a computer program product comprising one or more computer readable media having computer executable instructions for performing the steps of at least one of the aforementioned methods. Such a computer program product allows for efficiently performing the method according to the invention and achieving the respective benefits.

Among others, decomposing the data matrix using a factor decomposition method, such as PCA or Evolving Factor Analysis or other suitable method, allows for determining how many main solutions are needed to completely mimic the dynamic process or the specific process step or a variation thereof. Thereby, the factor decomposition methods can be used according to good modelling practices known by a person skilled in chemometrics.

Estimation of initial multicomponent analyte composition and/or relative amount for each main solution can, for example, be achieved by evolving factor analysis as, e.g., described in [2] or determination of the purest sample/variable as, e.g., described in [4], as described above. Particularly, such estimation can be performed as an initial estimation after determining the number of main solutions necessary to mimic the dynamic process or process step or variation thereof and eventually their appearance and disappearance time.

With the initial estimation and number of main solutions necessary to mimic the dynamic process or process step or variation thereof the data matrix D or its smaller parts $D_i$ as described below are deconvoluted by the MCR-ALS algorithm with imposed constraints to obtain and optimize the multicomponent analyte composition of each of the main solutions to be used and its relative amount at a specific time to mimic the dynamic process or process step or a variation thereof described by matrix D or $D_i$ if only part of the process is to be mimicked. Thus, deconvoluting the data matrix by a MCR-ALS algorithm with imposed constraints allows for mimicking the dynamic process or process step or a variation thereof described by the data matrix. In particular, the MCR-ALS constrained algorithm can resolve equation 1 mentioned above in an iterative way. Thereby, e.g., it can be implemented in the method according to the invention as follows: Firstly, establish the number of main solutions determined by a factor decomposition method, such as PCA or evolving factor analysis or other suitable methods. Secondly, estimate the initial multicomponent analyte composition (first iteration) of each main solution and/or the relative amount of each main solution. Thirdly, estimate the analyte concentration in main solution ($S^T$) based on logical constraints and physical/chemical constraints for main solutions (for example an analyte concentration cannot be negative - non-negative constraint). Fourthly, estimate the relative amount of each main solution (C) based on logical constraints and physical/chemical constraints (for instance non-negative constraint and the sum of all fractions should be equal to 1 - closure constraint). Fifthly, compare the results obtained by the MCR-ALS algorithm (C x $S^T$) with the original data matrix (D or $D_i$) using equations 2 through 4 mentioned above to evaluate the iteration performance. Sixthly, if the iteration performance is not as high as required then the initial estimation is changed and steps 3 to 5 described above are repeated. This will be repeated until the performance of the MCR-ALS algorithm is adequate to mimic the dynamic process or process step or a variation thereof presented in matrix D or $D_i$. Seventhly, when finished the analyte concentration for main solutions ($S^T$) that can mimic the dynamic process, process step or variation thereof or that can mimic part of it, and the amount of each main solution (C) at a given time of the dynamic process, process step or variation thereof are obtained for each of the experimental data points in D or $D_i$.

By obtaining a relative amount of each main solution (C) for the determined number of main solutions at a specific time each of the data points, i.e. the rows of the data matrix (D), can be related with a time variable. The specific time can be represented by a relative or absolute time variable from original process data. In particular, it can be any time between the beginning and the end of the dynamic process or process step or a variation thereof comprised in the data matrix. Like this, it is possible to represent the amount of each main solution (C) against the absolute or relative time variable (t) associated with each row of the data matrix D. This allows for increasing the number of simulated data points that can mimic the process or process step.

The MCR-ALS loading matrix also referred to as the $S^T$ matrix can particularly be an n x m matrix where n is the number of main solutions that, e.g., was established by a factor decomposition method, such as PCA, evolving factor analysis or other suitable method and m is the number of properties such as analytical composition, pH, temperature, etc. that has been measured in the dynamic process or the specific process step or a variation thereof and are included in the data matrix D, e.g. m is equal to the number of columns in matrix D, for the MCR-ALS algorithm. Thus, each row of $S^T$ is one main solution and each column of such row is a concentration or other property that follow the same order as the data matrix or D matrix. To obtain the main solutions of the MCR-ALS one may have to assemble the solutions so they can match the concentrations and properties established by the MCR-ALS algorithm, i.e., if main solution 1 ($S^T1$ - first row of $S^T$) results were concentration x for compound a, y for compound b and z for compound c, then one may have to determine the amount of each compound (x, y and z) so that the concentration of the solution is equal to the $S^T1$ vector. In a separate apparatus, $S^T2$ (second main solution and second row of the $S^T$ matrix) may have to be assembled according to the results obtained in the MCR-ALS algorithm and the same for the remaining main solutions. Such an apparatus, e.g., can be comprised in the synthetic sample preparing system described below.

Unlike the common use for MRC-ALS method or algorithm mentioned above, the method according to the invention uses MRC-ALS in a reverse way, i.e, it starts with the known profile such as, for instance, the composition of specific components along time, and finds the necessary main solutions and its relative amount in the mixture that allow to mimic dynamic processes or specific process steps or a variation thereof, assuming that dynamic processes or specific process steps or parts of such processes or a variation thereof can be simulated as a mixture of synthetic main solutions.

The method according to the invention can be carried out in a discrete, fed-batch or continuous manner with lower or higher level of automation. Although not limiting for the present invention, an apparatus for such experimental execution should comprise at least a mixing device of some kind and should allow the in-situ measurement of one or preferably more sensors such as, pH probes, temperature probes, spectroscopic sensors, and other analytical instruments that can be used to build multivariate calibrations and supervision systems. In a preferred embodiment this apparatus should also have at least temperature and pH control systems. And in a more preferred embodiment the apparatus should have the proper control systems that allow the recreation of the dynamic process conditions, or the specific process step conditions or a variation thereof that might influence the multivariable analytical devices used to build multivariate calibrations and supervision systems.

Thus, the present invention describes a new method that is comprised of creating a set of synthetic samples that mimic a dynamic process, a specific process step or a variation thereof that can be used to develop multivariate calibrations and supervision systems. In order to enhance calibration models accuracy and robustness the introduction of randomly or programmed spiked and orthogonal experimental designed samples is also described. The new method works by using prior knowledge of dynamic processes, a specific process step or variation thereof expressed by their available data such as, e.g., process parameters such as temperature, pH, feeds, etc. and quality control data such as analyte concentration, reagent and product concentration along time, etc. and using such data to build mixtures profiles that can be used to build synthetic samples that mimic the process dynamics. Like this, a novel methodology to overcome the calibration developments drawbacks listed above can be provided comprised by preparing synthetic multicomponent samples that can mimic dynamic processes, a specific process step or a variation thereof and using orthogonal design of experiments or random or programmed spikes or random or programmed mixing of samples to enhance calibration models performance.

The method described herein takes into account samples that mimic a process dynamic, i.e., have the same behaviour as process samples. Such a method can tackle problems related to process dynamics versus sampling frequency, allows the creation of a high number of samples such that several batches or operation modes can be mimicked, reduces the high amount of offline analytics needed (highly reduced since only main solutions have to be determined) and reduces time required to develop calibrations in complex systems (for instance fermentations). However, it does not solve the problem of highly correlated data present in the process.

Therefore, samples preferably are prepared according to an orthogonal design of experiments and spectroscopic or other analytical instruments data is acquired that can be related with chemical composition of physical properties of the samples determined by a reference analytical method. As such, within this step of the method a new set of samples is created that can break co-linearity between parameters or analytes present in the data matrix D. This can be achieved by preparing samples according to the orthogonal design of experiments, which may include full or fractional factorial designs or d-optimal designs, by random spiking of specific compounds in samples or by programmed spiking. The introduction of such samples allows the creation of correlation-breaking samples to enhance model robustness, accuracy and selectivity that are not attained by process mimicking. These samples may have to be prepared individually in the case of orthogonal designed samples or can be built from existing samples in the case of random or programmed samples. After sample preparation a multivariable analytical device can be used to acquire data (X) that can be related with chemical composition of physical properties of the samples determined by a reference analytical method (y).

As mentioned herebefore, random and programed spiking only account for known and measurable variations. In some cases, however, there might be some unknown or undesired correlations that have to be taken into consideration. Therefore, in order to break these correlations random or programed mixing of synthetic or process samples is recommendable. A detailed description can be found in example 4.

Thereby, synthetic multicomponent samples that mimic the dynamic process or the specific process step or a variation thereof and the prepared samples preferably are grouped together. In particular, the two data subsets, i.e. the synthetic multicomponent samples that mimic the dynamic process or the specific process step or a variation thereof and the co-linearity breaking samples, can be grouped together to produce accurate and robust multivariate calibrations for each of component following good modelling practices known by persons skilled in chemometrics. For the development of a supervision system based on MSPC control charts and graphics, only the synthetic multicomponent samples that mimic the dynamic process or the specific process step or a variation thereof are used.

In summary, the method according to the invention is comprised of a series of steps that are described in detail above and below. According to the invention, it is assumed that dynamic processes or specific process step or a variation thereof can be mimicked by the use of scale independent multicomponent mixtures, hereafter and herebefore called main solutions, and by adding different amounts of such solutions in a mixture, hereafter and herebefore called relative amount of solution.

The described method allows to significantly reduce time and resources to develop multivariate calibrations and supervision systems based on spectroscopic sensors by allowing scale down from process scale to lab scale, reducing the amount of reference analytics and decreasing the amount of time for creating the dataset to be used in such multivariate calibration and supervision systems. Furthermore, the multicomponent systems developed by the presented method enable the estimation of chemical (concentration, relative amounts, acidity, etc.), physical (e.g. state of cells, density, etc.) properties and multivariate process profiles based on spectroscopic sensors.

With the described method, process dynamics can be followed, e.g. reaction profiles, mixing profiles, where profile is considered to be a trend that evolves with time and samples can be produced that follow such a trend. As such, the invention allows to build evolving (Batch) multivariate statistical control charts (also many times referred as MSPC) using the spectra registered on the produced samples and by mixing 2 or more of this samples to produce sub-samples that mimic a process trend that evolves with time.

With the present invention, samples and sub-samples can be created that can mimic the spectral information of a reaction without having to execute a real reaction.

Using the described method and/or system, mathematical or computational simulation of spectra to develop the calibration can be avoided. In fact, real complex samples are created that mimic process trends (for instance, reaction profiles) from which spectra are acquired. As the method allows creating samples that mimic process evolution as a function of time, the user can build much more samples than using the methods disclosed in the prior art and can benefit from the reduced amount of analytical burden.

Furthermore, as the method allows for chemical and physical variation to be mimicked, there is no need to simulate the spectra of the process by computational/mathematical simulations. Also the described method allows to measure spectral non-linearity imposed by processes and also takes into account other interferences (for instance, one could use a different media in a fermentation run to introduce such variation in the process without having to execute a fermentation run). Finally, spectra acquired from real samples are used and not simulated by an algorithm or computational method. Thus, the method allows the usage of the matrix effects in the presented model.

Preferably, matrix effects in this context are changes in the spectra that cannot be related with changes in the process state (i.e. its composition at a given time). Preferably, matrix effects can be one of the following:

1) Aeration increases the number of bubbles in fermentations while the composition is kept constant. This will change a NIRS measurement but it is not related with the chemical composition.

2) Some of the chemical compounds are not possible to measure in a process sample and there is no process history available about them but a change in MIR spectra is detected. This is also cofounded as a matrix effect.

3) Composition of fermenter volume is changed by the feeds but the analytical data does not show it (minor changes, complex composition can hardly or not be measured, etc.). It is also considered as a matrix effect.

One of the advantages of the invention is that matrix effects can be taken into account by using samples from the process and random mixing can be performed so that these unwanted effects can be removed from the model, or at least taken into account, i.e. increasing model robustness.

Moreover, the method allows to determine how many main solutions, are required to mimic a process (for instance a fermentation run), which is the chemical composition of each main solution and how to mix such solutions in order to obtain the synthetic samples that mimic the process or process step.

In summary, the method and the system according to the invention allow to mimic processes and their variation by assembling the main solutions by weighting each of the chemical compounds or measuring a volume of a dissolved compound or mixture and diluting them in a solvent (for instance media) to obtain the concentrations determined by the method according to the invention and then mixing the required volumes of each main solution to obtain synthetic samples that mimic process samples.

Also, since this method allows the process to be mimicked as a mixture of solutions instead of a real process step, it is possible to make the sampling frequency adequate for process dynamics. This can be an advantage over developed calibrations and supervision systems based only on process samples since this allows the development of evenly distributed and more robust calibrations.

Additionally, because this method allows the recreation of samples/profiles based on historical data at lab scale and since historical data is largely available in the industry this allows the development of very specific quality by design calibrations and supervision systems that can include the entire design space of samples from conception, through process development and industrial production.

Synthetic samples produced in this way can have the same high correlation coefficients between some of the parameters as real in-process samples, which do not guarantee spectral selectivity and non-correlativity requisites for calibration development. As such, the use of this method can be combined with orthogonal experimental designed or randomly/programmed spiked samples as proposed in the present invention.

The use of such a mixed model allows combining advantages of both methods and allows the creation of accurate and robust multivariate calibrations and supervision systems based on spectroscopic sensors and chemometrics.

These and other aspects of the invention will be apparent from and elucidated with reference to the embodiments and examples described hereinafter.

Summarizing the above, the invention relates to a method to tackle the drawbacks that are usually found in the development of multivariate calibrations and monitoring systems based on spectroscopic measurements (near infrared, mid infrared, Raman, 2D-Fluorescence and other spectroscopies) outlined above.

To overcome these drawbacks it is proposed to create two different types of multicomponent samples (synthetic multicomponent samples and co-linearity breaking samples). Using both groups of samples for multivariate calibrations assures better accuracy and robustness and is an innovative approach in this scientific area because one does not need to collect real process samples.

Thereby, the main achievements are:

- Reduction of time (14 days of fermentation can be reduced to two days of work), analytical and human resources necessary to develop Multivariate Calibrations and Supervisory Monitoring and Control Systems.
- Simulation of fermentations and spectra acquisition can be done in laboratory scale (scale down of industrial dynamic processes, specific process step or variation thereof).
- Better description of process dynamics by creating adequate sample frequency (creating more samples for parts of the process with increased dynamics).
- The use of a mixed model guarantees the best of both methods, allowing the creation of accurate and robust multivariate calibrations and supervision systems of dynamic processes, or specific process step or a variation thereof, in which fermentations are included.

It is described above and below that the creation of synthetic multicomponent samples that uses several known chemometric methods/algorithms, integrated into an innovative methodology to mimic dynamic processes or a specific process step or a variation thereof. Also, a take on a specific chemometric method allows the creation of these samples in an innovative way.

An aspect which might be of particular relevance in certain scenarios is the creation of supervisory systems based on the methodology proposed. E.g., Example 6 below illustrates one possible supervision system created in accordance with the present invention. This point specifically can open a very important field of scaling up (potentially speeding up industrialization of biopharmaceutical processes).

Also the creation of co-linearity breaking samples is explained. The use of both types of multicomponent samples to develop multivariate calibrations might be of particular relevance. Example 5 below illustrates one possible accurate and robust multivariate calibration created in accordance with the present invention.

Brief Description of the Drawings

The method, computer program and system according to the invention are described in more detail herein below by way of exemplary embodiments and with reference to the attached drawings, in which:

Fig. 1    shows analyte corridors during fermentation and MCR model wherein the squares ($\square$) represent off-line data and the connected circles (-●-) represent MCR-ALS model or algorithm of a first example (Example 1) of the invention;

Fig. 2    shows solution A1 fraction profile (●) in data subset 1 and the $2^{nd}$ order polynomial interpolation (-) of a second example (Example 2) of the invention;

Fig. 3    shows analyte profiles for MCR based planning data (black points - ●) and experimental data (diamonds - ◊) in a total of 63 runs of the second example of the invention;

Fig. 4    shows linear correlation between Glutamate (GLU) and Product (PRO) (Spk = spiking) of a third example (Example 3) of the invention;

Fig. 5    shows in section A NIRS viable cell density model performance with 1 LV of a fifth example (Example 5) of the invention and in section B MIRS glucose model performance with 4 LV using samples created using the present invention method of the fifth example wherein black points (●) represents calibration data and diamonds (◊) represents external validation data, while dotted line (- - -) indicates 1:1 diagonal;

Fig. 6    shows in section A on-line monitoring of a fermentation run predicted with NIRS VCD model from the fifth example wherein black points (●) represent on-line prediction of fermentation run and diamonds (◊) represent off-line reference analytics and in section B on-line monitoring of fermentation runs predicted with MIRS glucose model from the fifth example wherein black points (●) represent on-line prediction of fermentation run and diamonds (◊) represent off-line reference analytics;

Fig. 7    shows multivariate batch profiles of a fermentation run (black points - ●) and synthetic multicomponent samples (circles - ○) mimicking a historical fermentation profile (circles - ○) of a sixth example (Example 6) of the invention wherein in section A PC1 scores profile is shown and in section B PC2 scores profile; and

Fig.    8 shows a diagram according to an embodiment of the invention.

Mode(s) for Carrying Out the Invention

In the following, embodiments of the invention are described by means of specific examples. The examples are provided for illustrative proposes, and are not intended to limit the scope of the invention as claimed herein. Any variations in the exemplified articles are intended to fall within the scope of the present invention.

Example 1

Example 1 relates to synthetic multicomponent fermentation samples. It is intended to show a step-by-step implementation of part of the present invention in a multicomponent (several analytes) complex system, without imposing any limitations on its use in other systems not covered by this example.
A good example for the application of the present invention is its use to mimic fed batch fermentations, as for example, Chinese Hamster Ovary (CHO) mammalian cell cultivation. Such fed batch fermentations contain multicomponent complex matrix analytes that vary depending on cell activity and imposed process conditions such as feeds, feed rates, pH and temperature profiles, etc..
These systems are rather complex in its nature and the need of their monitoring has long been an integral part of industrial processes since it allows increased automation and introduction of advanced control schemes. In recent years, process analytical technology (PAT) has emerged as a drive motor for overcoming the drawbacks of process monitoring, generally related with lack of capturing processes dynamics by insufficient sampling frequency and the high amount of offline analytical measurements needed at the process that are not available at the right time (time between sampling and analysis is too long for process control).
Applying PAT tools to real-time multivariate data collected by an on-line sensor (such as Near Infrared, Mid Infrared, 2D Fluorescence, Dielectric Spectroscopies, or any other kind of on-line sensors) provides an efficient means of identifying/reducing variation, managing process risks, relating process information to critical quality attributes (CQAs) and determining process improvement opportunities such as detecting contaminations, increasing yield, reducing impurities variability and implementing advance process control for such processes. Although their big advantages, PAT tools require a development stage, also called calibration, where in-process samples have to be measured by the on-line sensor and a sample can be withdrawn to be analyzed by standard analytical methods, although in some cases this analysis is not required. Usually, in-process samples have to be measured in process conditions, but the following example shows a way to create such multicomponent synthetic samples that mimic the process dynamics without the need to collect data in a real process.
In this example, the analytes profiles from a typical fed-batch fermentation were measured by offline standard analytical methods and recorded in digital format to make them available for future reference of batch trends. For exemplification of the present invention one culture parameter (viable cell density) and seven analytes profiles (glutamine, glutamate, glucose, lactate, ammonia, product and asparagine) were chosen to recreate such a fermentation using synthetic multicomponent mixtures.
*(Step* 1) The first step is the evaluation/organization of such historical process data. In the present example this dataset contains analytical data for viable cell density (VCD), glutamine (GTF), glutamate (GLU), glucose (GLF), lactate (LAF), ammonia (NHF), product (PRO) and aspargine (ASN), as well as the fermentation time (t) at which the sample was obtained from the fermentation batch. For this example no physical parameters were considered as the fermentation was run under constant pH and temperatures. Table 1 shows the normalized data matrix considered for this example already ordered according to the requirements presented for the present invention.
(Step 2) In a second step, the data matrix D is created from the data available in Table 1. Columns 2 to 9 (VCD to ASN) are used, yielding a D matrix with 14 rows (samples) and 8 columns (multicomponent analyte composition), which is then pre-processed using an auto scaling data pre-treatment. The resulting data matrix is then analyzed by Principal Component Analysis to estimate the number of main solutions needed to mimic the process dynamics presented in Table 1. In this case, no other pre-processing technique of the dataset was needed, but in some cases the use of other pre-processing algorithms might be recommended.

Table 1: Normalized Historical Process Data Used to Create Synthetic Multicomponent Fermentation Process Samples

| t | VCD | GTF | GLU | GLF | LAF | NHF | PRO | ASN |
|---|---|---|---|---|---|---|---|---|
| 0.06 | 0.04 | 1.00 | 0.33 | 0.75 | 0.06 | 0.10 | 0.00 | 0.97 |
| 0.13 | 0.09 | 0.85 | 0.36 | 0.74 | 0.13 | 0.18 | 0.00 | 0.95 |
| 0.20 | 0.18 | 0.61 | 0.39 | 0.68 | 0.23 | 0.31 | 0.01 | 0.85 |
| 0.28 | 0.36 | 0.27 | 0.41 | 0.56 | 0.48 | 0.43 | 0.03 | 0.68 |
| 0.35 | 0.57 | 0.08 | 0.43 | 0.31 | 0.66 | 0.44 | 0.06 | 0.46 |
| 0.44 | 0.90 | 0.05 | 0.41 | 0.77 | 0.61 | 0.34 | 0.14 | 0.18 |
| 0.51 | 0.96 | 0.08 | 0.46 | 0.86 | 0.42 | 0.27 | 0.22 | 0.27 |
| 0.58 | 0.96 | 0.11 | 0.53 | 0.91 | 0.20 | 0.35 | 0.31 | 0.32 |
| 0.65 | 0.90 | 0.14 | 0.62 | 0.94 | 0.16 | 0.46 | 0.41 | 0.35 |
| 0.72 | 0.76 | 0.16 | 0.70 | 0.96 | 0.18 | 0.58 | 0.51 | 0.39 |
| 0.78 | 0.71 | 0.19 | 0.78 | 0.94 | 0.29 | 0.65 | 0.59 | 0.42 |
| 0.86 | 0.71 | 0.20 | 0.85 | 0.92 | 0.50 | 0.73 | 0.67 | 0.45 |
| 0.92 | 0.63 | 0.21 | 0.90 | 0.89 | 0.76 | 0.83 | 0.73 | 0.49 |
| 1.00 | 0.54 | 0.21 | 0.98 | 0.81 | 0.97 | 1.00 | 0.79 | 0.53 |

The pre-processed matrix data **D** from step two was decomposed using the Principal Component Analysis. This allows obtaining the optimum number of multivariate main solutions that have to be used in the present invention to mimic the dynamic process or process step or a variation thereof. For selecting the optimum number of components the usual good modelling practices for PCA was used, i.e., the number of components should guarantee capturing more than 95% of data variation, only principal components with eigenvalue higher than 1 should be chosen. As such, for the present example three principal components were chosen as can be seen in Table 2. This number of components is equal to the initial estimate of the number of main solutions necessary to describe the fermentation batch profile with 97.4% of the data captured by the model.

Table 2: Principal Component Analysis results for the pre-processed matrix data D

| Principal Component Number | Eigenvalue | % Variance Captured by this PC | % Variance Captured (TOTAL) |
|---|---|---|---|
| 1 | 4.7 | 58.5 | 58.5 |
| 2 | 1.7 | 21.5 | 80.0 |
| 3 | 1.4 | 17.4 | 97.4 |

As a result of the PCA, three main solutions can be used to prepare the synthetic multicomponent samples to mimic the process dynamics. Although this number is not too high there might be limitations in the implementation of such a procedure. As such, the present example will assume that it was only possible to use binary mixtures of main solutions to mimic the entire fermentation or parts of it. As it can be seen from Table 2, using only two components to describe the complete fermentation run leads to a large amount of variation that is not explained (up to 20%) by the PCA, resulting in deviations from the original data (batch profile for each analyte). As such, in this case (explained variation < 95%) it is advisable to split the dynamic process (fermentation) into smaller fractions of such process. For illustrative purposes, the present example will use only two main solutions to describe parts of a complete dynamic process. In the end, to simulate the complete batch run of the present examples it is necessary to run each of its smaller parts and group the data sets together at the end.

To obtain the smaller parts of the fermentation process that can be mimicked using only two main solutions, matrix D was reanalyzed using PCA algorithm but this time starting with a smaller number of samples (three to start with), alternatively an Evolving Factor Analysis can be used to identify changes in the dynamic process in a more automatic way. The three samples were analyzed by fixing the number of PCs to two and if the explained variance was higher than 95% then another sample was added to the data set. This procedure was stopped when the captured variance fell below 95%. At this time, the n-1 iteration is considered as the best interval for the first subset of the original **D** matrix. In order to maintain continuity for the fermentation profile, the second subset of data starts at the same point that the preceding subset ends. The procedure is repeated to find the second, third and other necessary intervals until the complete fermentation run is covered. The results obtained with such procedure for the present example contains four data subsets, which are presented in Table 3.

Table 3: Data subsets necessary to describe the fermentation run using only two main solutions

| Data Subset | Normalized Fermentation time |
|---|---|
| 1 | 0.06 - 0.35 |
| 2 | 0.35 - 0.51 |
| 3 | 0.51 - 0.65 |
| 4 | 0.65 - 1.00 |

*(Step 3)* After establishing the number of main solutions for each part of the fermentation run the MCR-ALS algorithm included in this invention requires an initial estimation of the multicomponent analyte composition and relative amount of each main solution. The determination of the purest sample was used for initial estimation based on [4]. This estimation assumes that the initial content of main solution is as close as possible to 1 (or 100%) while the other main solution is as close as possible to 0 (or 0%).

*(Step 4)* With the number of main solutions fixed by the PCA procedure and the initial estimation set the MCR-ALS algorithm can be used to extract the main solutions composition and their mixture profile necessary to mimic the fermentation batch run. In order to obtain meaningful solutions, three logical constraints were used:

- Non-negativity constraint for the fractions (or percent) of each solution, i.e., the relative amount of each solution has to be zero or higher;
- Non-negativity constraint for the analyte concentration and cell parameter in each solution, i.e., the amount of each analyte or cell parameter has to be equal or higher than zero;
- Closure constraint for the relative amount of each main solution, i.e., the sum of the relative amounts for main solution 1 and 2 is equal to 1 (or 100%) of the fermenter solution.

*(Step 5)* The results from MCR-ALS algorithm for each of the data subsets were obtained and are presented in Table 4 and 5. The relative amounts of each main solution are described by the scores of the MCR-ALS algorithm (Table 4) whereas the multicomponent analyte composition of each main solution is described by the loadings of the MCR-ALS algorithm (Table 5).

Table 4: Relative amount of solution to mimic fermentation run batch

| Data Subset i | Fermentation Time | Relative Amount of Main Solution Ai | Relative Amount of Main Solution Bi |
|---|---|---|---|
| 1 | 0.06 | 1.00 | 0.00 |
|  | 0.13 | 0.90 | 0.10 |
|  | 0.20 | 0.74 | 0.26 |
|  | 0.28 | 0.38 | 0.62 |
|  | 0.35 | 0.00 | 1.00 |
| 2 | 0.35 | 0.00 | 1.00 |
|  | 0.44 | 0.63 | 0.37 |
|  | 0.51 | 1.00 | 0.00 |
| 3 | 0.51 | 0.51 | 0.49 |
|  | 0.58 | 0.84 | 0.16 |
|  | 0.65 | 0.94 | 0.06 |

(continued)

| Data Subset i | Fermentation Time | Relative Amount of Main Solution Ai | Relative Amount of Main Solution Bi |
|---|---|---|---|
| 4 | 0.65 | 1.00 | 0.00 |
| | 0.72 | 0.96 | 0.04 |
| | 0.78 | 0.81 | 0.19 |
| | 0.86 | 0.57 | 0.43 |
| | 0.92 | 0.30 | 0.70 |
| | 1.00 | 0.00 | 1.00 |

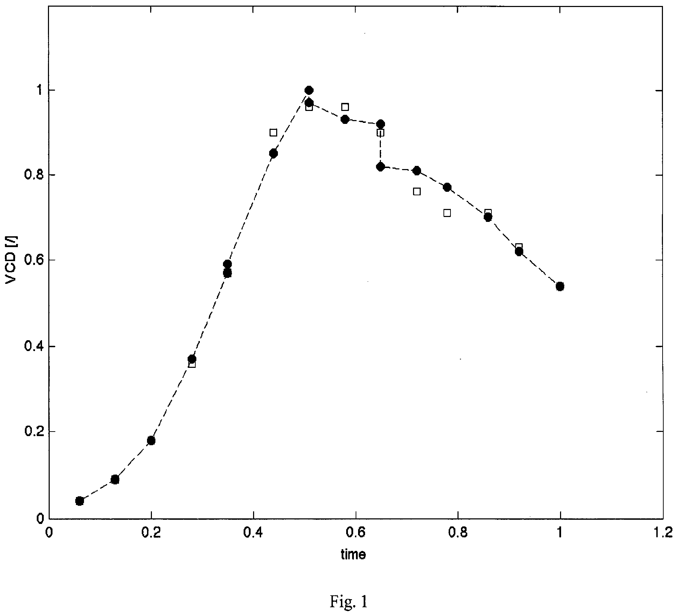

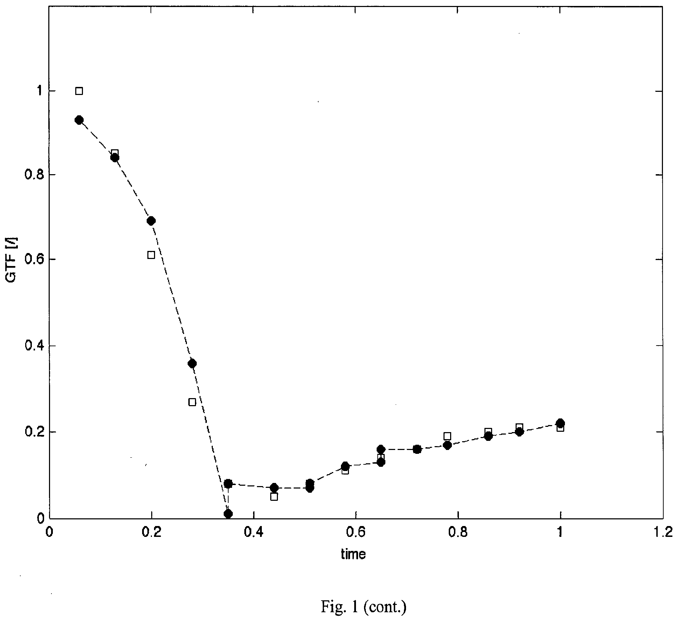

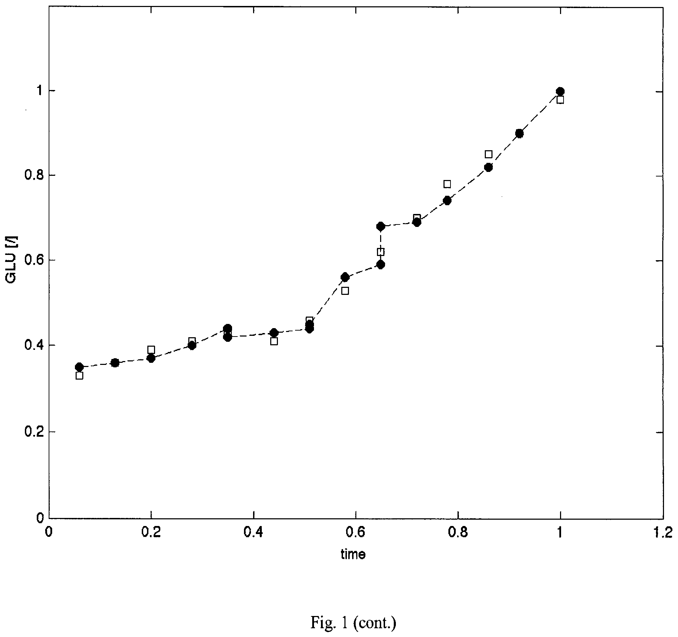

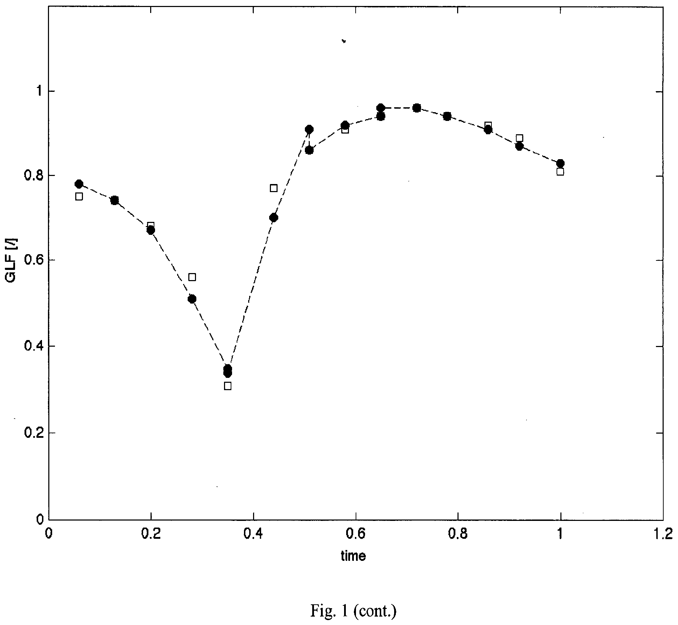

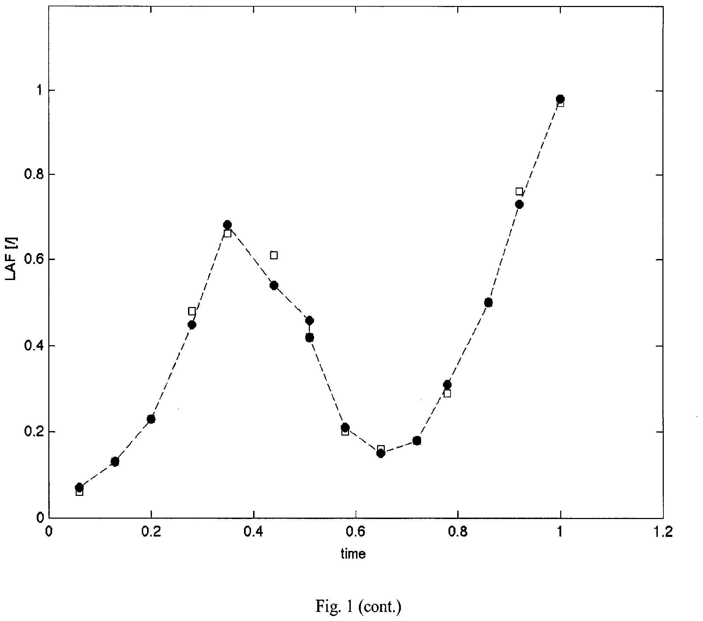

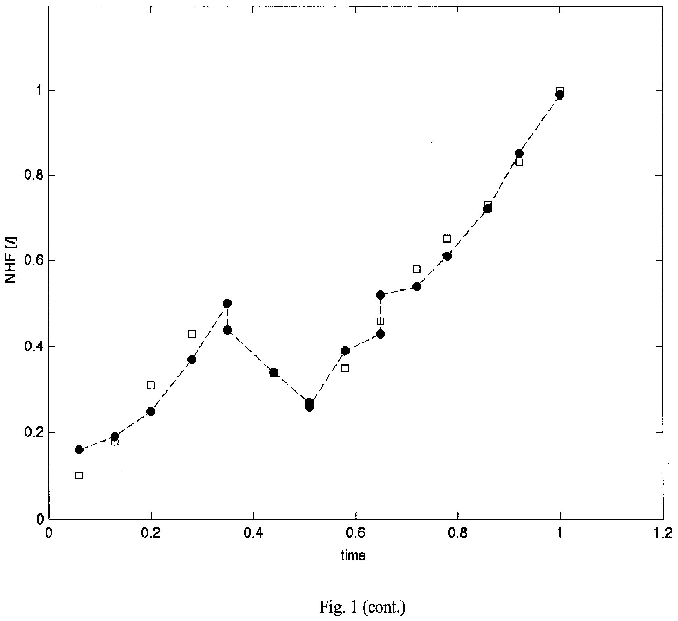

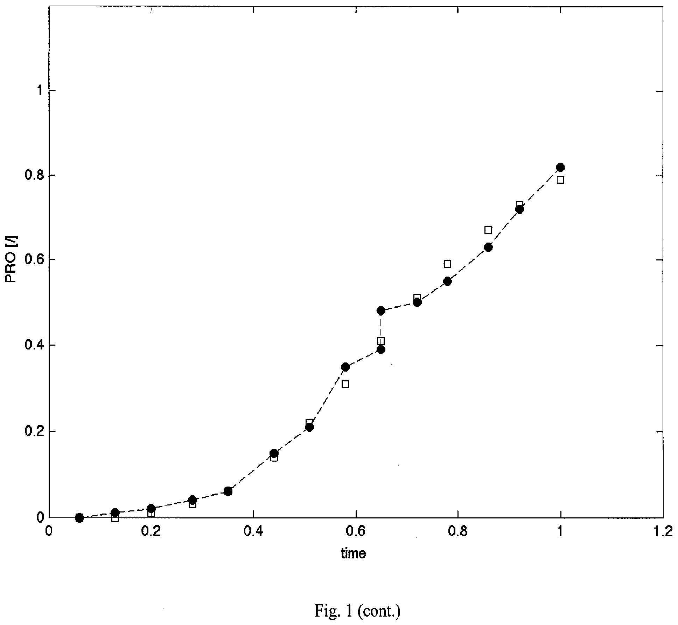

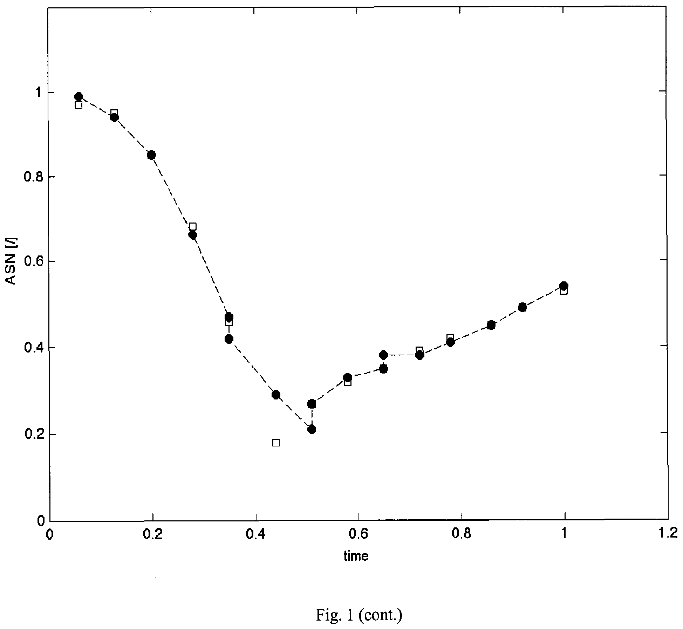

*(Step* 6) The MCR-ALS algorithm estimations were evaluated by plotting the batch profiles of each analyte contained in the original data (matrix **D**) and MCR-ALS estimations obtained by multiplying the relative amount of solutions (Table 4) by the concentration of analytes in the main solutions (Table 5), as described in Equation 1 above. As it can be seen in Fig. 1, the MCR-ALS models performance is very similar to the off-line data used to generate such main solutions and their fractions. Thus, MCR-ALS algorithm can be used to mimic dynamic processes, as the one described in the present example.

Table 5: Normalized concentration of analytes in main solutions

| Solutions | VCD | GTF | GLU | GLF | LAF | NHF | PRO | ASN |
|---|---|---|---|---|---|---|---|---|
| A1 | 0.04 | 0.93 | 0.35 | 0.78 | 0.07 | 0.16 | 0.00 | 0.99 |
| B1 | 0.57 | 0.01 | 0.44 | 0.35 | 0.68 | 0.50 | 0.06 | 0.47 |
| A2 | 1.00 | 0.07 | 0.44 | 0.91 | 0.46 | 0.27 | 0.21 | 0.21 |
| B2 | 0.59 | 0.08 | 0.42 | 0.34 | 0.68 | 0.44 | 0.06 | 0.42 |
| A3 | 0.91 | 0.14 | 0.62 | 0.95 | 0.11 | 0.45 | 0.41 | 0.36 |
| B3 | 1.03 | 0.02 | 0.28 | 0.77 | 0.73 | 0.07 | 0.00 | 0.18 |
| A4 | 0.82 | 0.16 | 0.68 | 0.96 | 0.15 | 0.52 | 0.48 | 0.38 |
| B4 | 0.54 | 0.22 | 1.00 | 0.83 | 0.98 | 0.99 | 0.82 | 0.54 |

The resolved concentration profiles for each simulated mixture is compared with the original experimental dataset (Fig. 1), proving that the present invention can be used to mimic complex multicomponent processes. Furthermore, the use of statistical measurements to evaluate the MCR-ALS model (Table 6) shows that the model accurately predicts the analyte concentrations showing RMSEP values that are of the same order as the errors associated to offline techniques.

Table 6: RMSEP and standard deviation of analytical methods for normalized analytes and culture parameter predicted by MCR-ALS models

| Analytes | RMSEP | SEL/ σ | Units |
|---|---|---|---|
| Viable Cell Density | 0.031 | 0.047 | a.u. |
| Glutamine | 0.039 | 0.025 | a.u. |
| Glutamate | 0.025 | 0.024 | a.u. |
| Glucose | 0.029 | 0.010 | a.u. |
| Lactose | 0.024 | 0.009 | a.u. |
| Ammonia | 0.038 | 0.060 | a.u. |
| Product | 0.027 | 0.003 | a.u. |
| Asparagine | 0.033 | 0.030 | a.u. |

Example 2

Example 2 relates to increasing the number of Synthetic Multicomponent Fermentation Samples for better capturing of process dynamics.

One of the drawbacks of off-line measuments in dynamic processes is the low amount of samples available to measure when compared to the variation of specific analyte.

This is of great importance when dealing with online sensors, such as Near and Mid Infrared spectroscopies, among other sensors. Thus, the present example uses part of the solution provided in Example 1 to increase the number of samples that can be used to mimic process steps. This is of great use if a very defined profile is necessary in some parts of the process, specially to increase the number of samples in undersampled process phases. Good examples of such under sampling can be seen in GTF profile in Fig. 1. As it can be seen, the main variation of data for this analyte is limited for the first data subset interval (from 0.06 to 0.35 time units) and if the measurements were made using only the available data the number of samples would be reduced to 5 in Example 1. As such, the creation of sample between 0.06 and 0.35 time units can help in developing calibrations for online sensors as it guaranties that the transition is better captured and that the number of samples with variation is uniform.

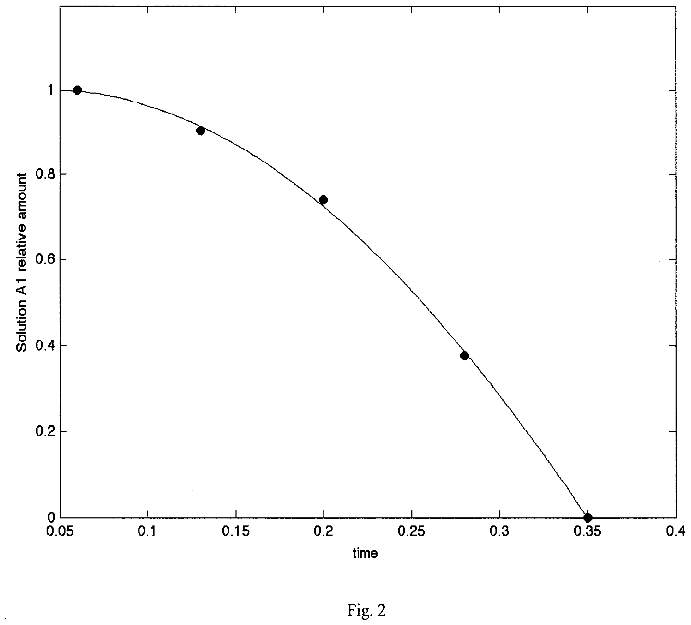

Using the obtained main solutions relative amount on data subset 1 and the corresponding fermentation times (in time units) from the original data matrix D, it is possible to represent each main solution relative amount as a "relative amount profile", i.e., relative amount represented against time (Fig. 2). From that representation it is possible to obtain a $2^{nd}$ order polynomial that can be used to estimate the amount of this solution for a specific time. As so, to increase the number of samples it is only needed to establish the fermentation times required to be mimic. Since this is a binary system and the total amount of the mixture fraction has to be 1, the amount of solution B1 can be calculated as a difference from 1 and the relative amount of A1 solution.

By using the method described in this example the number of samples can be increased according to the users needs. Furthermore, this is not limited to a binary system and can be used to increase the number of samples in more complex systems, where the number of solutions is higher.

The increase of samples was also done to other data subsets presented in example 1 of the present invention. The MRC-ALS data and the increased data points obtained in this step of the invention were used to create the synthetic samples that mimic the historical fermentation batch. An example of the relative amounts of each solution for the first data subset (solutions A1 and B1) is presented in Table 7.

Table 7: Relative amount of solution to mimic fermentation run batch

| Data Subset i | Fermentation Time | Relative Amount of Main Solution Ai | Relative Amount of Main Solution Bi |
|---|---|---|---|
| 1 | 0.06 | 1.00 | 0.00 |
| | 0.09 | 0.97 | 0.03 |
| | 0.12 | 0.93 | 0.07 |
| | 0.15 | 0.87 | 0.13 |
| | 0.18 | 0.78 | 0.22 |
| | 0.21 | 0.69 | 0.31 |
| | 0.24 | 0.57 | 0.43 |
| | 0.27 | 0.43 | 0.57 |
| | 0.30 | 0.28 | 0.72 |
| | 0.33 | 0.11 | 0.89 |
| | 0.35 | 0.00 | 1.00 |

This process was extended to the other data subsets presented in Example 1 and the method was tested experimentally by assembling the solutions (A1, A2, A3, A4, B1, B2, B3 and B4) and recreating the process dynamic by mixing the determined amount of each main solution for that specific time.

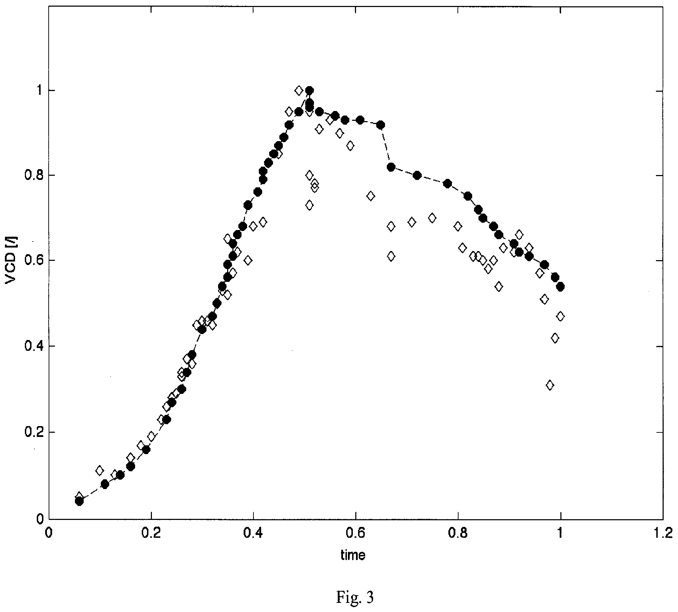

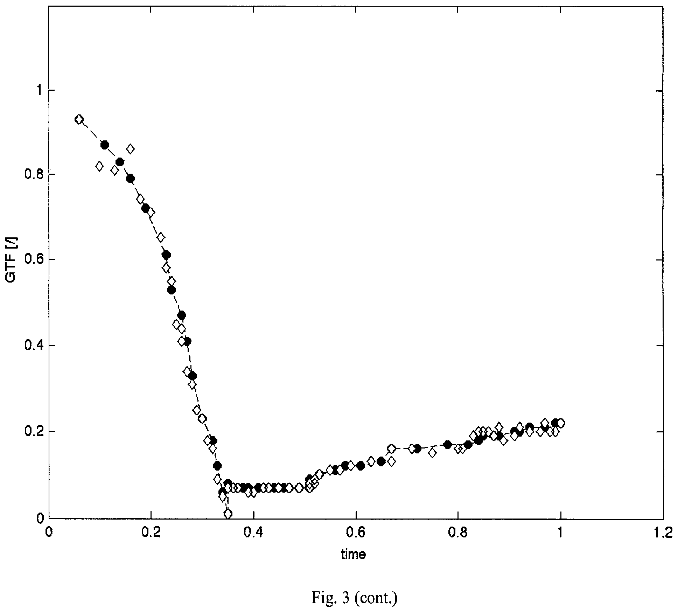

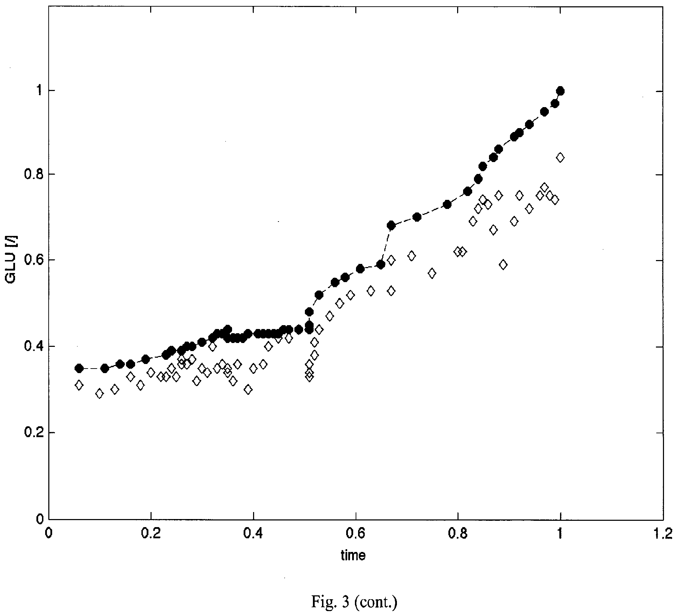

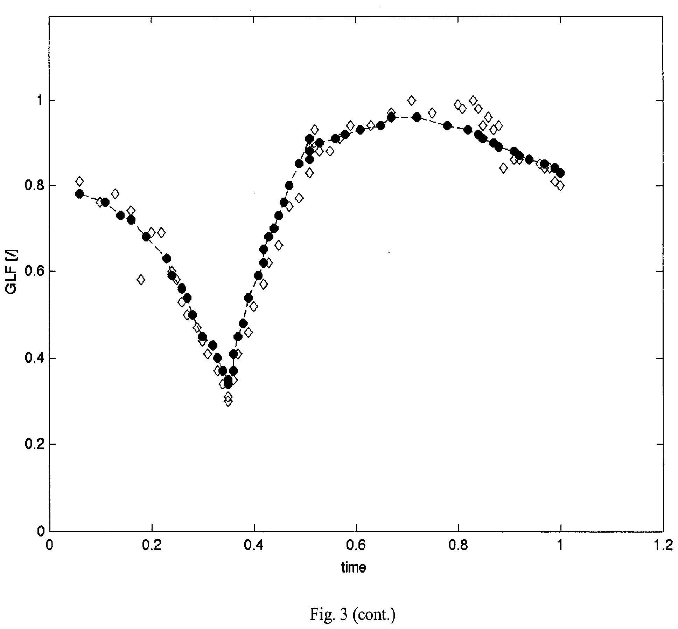

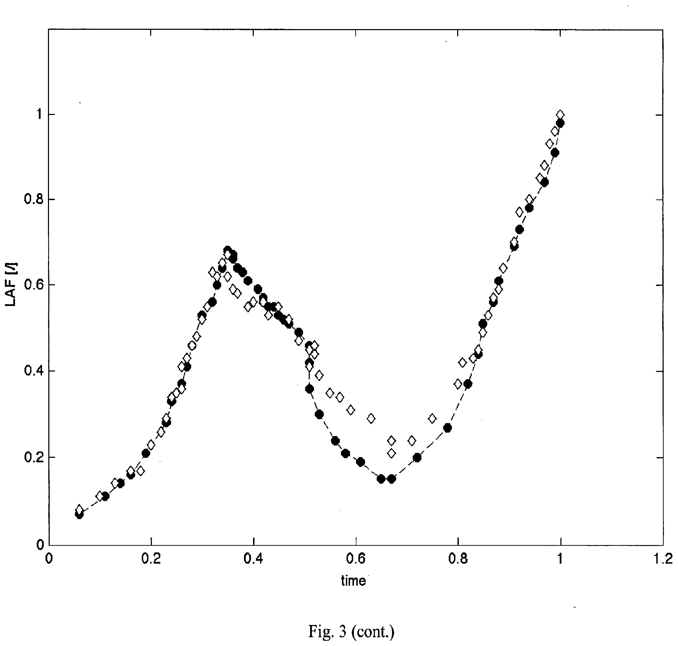

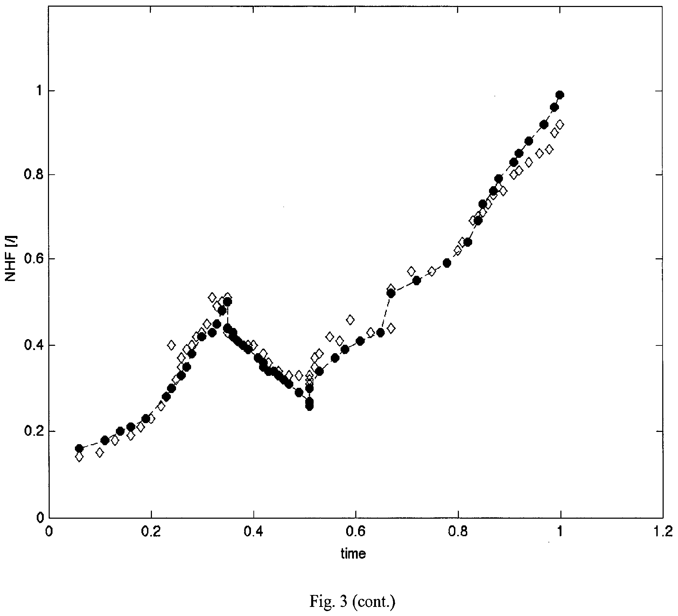

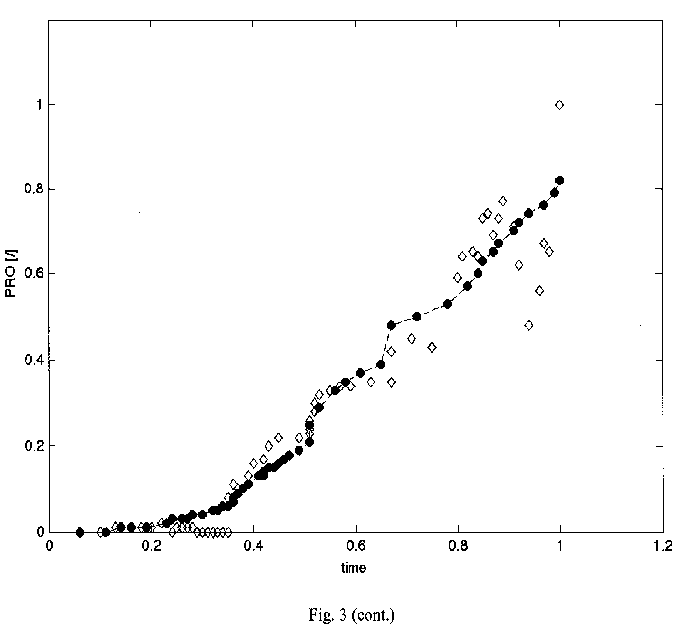

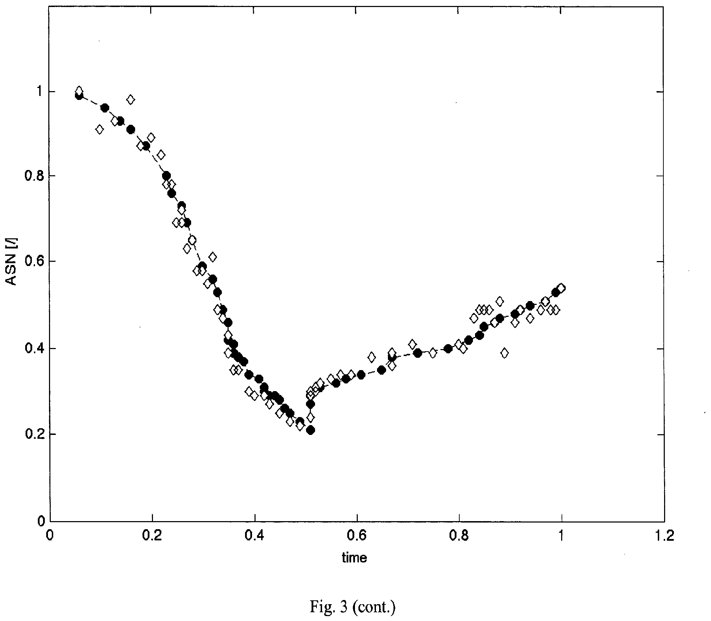

The experimental implementation was evaluated by plotting the batch profiles of each analyte contained in the original data (matrix **D**) and the profiles obtained by creating the samples described above. As it can be seen in Fig. 3, the samples generated by this method recreate the analyte profile of the fermentation batch. Thus, this method can be used to mimic dynamic processes, as well as increase the number of samples from the original data set (matrix **D**).

Example 3

Example 3 relates to creating data samples to make calibration models more accurate and robust.

The examples presented so far only represents the process dynamics, and as such, have the same behaviour as process samples. The fermentation profiles naturally retain a metabolism-induced concentration correlation between cellular substrates and metabolic products. In fact, analyzing the correlation of the analytes presented in the off-line data (Table 1) show that these correlations are evident. Since the data generated by Example 1 and Example 2 completely mimics the fermentation profile it is also expected that the correlations are still retained by those synthetic multicomponent samples. As it can be seen in Table 8 some analytes and culture parameters present high correlation (0.8<|R|<1), medium correlation (0.6<|R|<0.8) or slight correlation (0.5<|R|<0.6), while others present no correlation at all (|R|<0.5). From those the analytes and culture parameter profiles that are highly correlated, may lead to calibrations with lack of selectivity, thus creating indirect calibrations that might underperform if the new batches present different profiles. Thus, the present invention comprises a step of creating a new set of samples that can break co-linearity between parameters or analytes present in the data matrix D, enabling the creation of samples to be included in the development of robust and accurate calibrations of spectroscopic sensors.

|  | VCD | GTF | GLU | GLF | LAF | NHF | PRO | ASN |
|---|---|---|---|---|---|---|---|---|
| **VCD** | 1.00 |  |  |  |  |  |  |  |
| **GTF** | -0.89 | 1.00 |  |  |  |  |  |  |
| **GLU** | 0.37 | -0.43 | 1.00 |  |  |  |  |  |
| **GLF** | 0.45 | -0.15 | 0.53 | 1.00 |  |  |  |  |
| **LAF** | 0.22 | -0.50 | 0.52 | -0.24 | 1.00 |  |  |  |
| **NHF** | 0.31 | -0.51 | 0.95 | 0.28 | 0.70 | 1.00 |  |  |
| **PRO** | 0.47 | -0.48 | 0.99 | 0.62 | 0.46 | 0.90 | 1.00 |  |
| **ASN** | -0.98 | 0.94 | -0.35 | -0.33 | -0.34 | -0.34 | -0.44 | 1.00 |

Table 8: Data correlation analysis map for samples generated with examples 1 and 2. Shaded Grey – autocorrelation (R=1), High correlation (0.8<|R|<1), Medium correlated (0.6<|R|<0.8), Slightly correlated (0.5<|R|<0.6), low or no correlation (0<|R|<0.5).

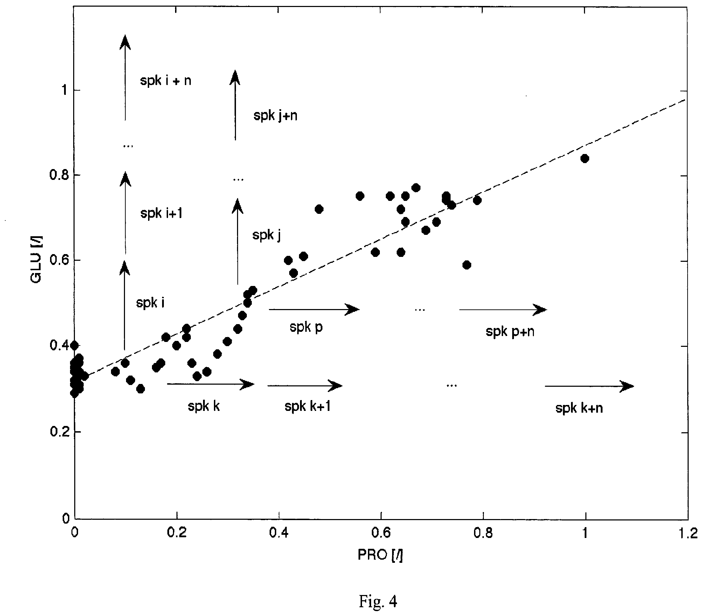

The preparation of such samples can be done in several ways, including, but not limited to, orthogonal design of experiments, random spiking of specific compounds in samples or by programmed spiking. The present example illustrates a way of implementing programmed spiking to break co-linearity of fermentations profiles. These programmed spiking samples include looking at Table 8 and searching for pairs of analytes that present a high correlation, for instance the correlation between product (PRO) and Glutamate (GLU). As is can be seen in Fig. 4, this linear correlation can be presented in a plot of the amount of Glutamate (GLU) against the amount of product (PRO).

A way to break such correlations is to use samples that maintain the linear correlation and spike them with one of the analytes, maintaining the other as a constant value. This procedure will break the correlations between a pair of analytes. The introduction of such spikes creates a new sample that starts to break such correlations (showed in Fig. 4 as Spk1) shown by the linear trend. As it can be seen in Fig. 4, increasing the number of spikes can produce samples that are not available from the fermentation run, as well as samples that completely cover the design space of the GLU vs. PRO combinations, i.e., samples with low content of GLU and high content of PRO and samples with high content of GLU and low content of PRO. Also, this procedure can be done in several levels of Fig. 4, i.e., it is not necessary that all samples are to be prepared from samples with low level of GLU and PRO, for instance, some samples can be prepared in the same way by spiking known amounts of each analyte on samples with medium values of GLU and PRO (GLU and PRO within the 500 to 600 range) or even with higher values of GLU and PRO.

In order to achieve an optimal dataset for calibration development, this analysis and sample preparation procedure can be repeated for other pairs of variables identified in Table 8 as having high and medium correlation.

The samples created with this procedure can be added to the dataset of samples created in Example 1 and Example 2, creating an extended dataset that has lower correlations as its characteristics. As it can be seen in Table 9, the inclusion of such samples in the dataset created in Example 1 and Example 2 allows the construction of correlation-breaking samples to enhance model robustness, accuracy and selectivity that are not attained by process mimicking. This is confirmed by the reduction of data correlation between each pair of analytes (presented in Table 9).

| | VCD | GTF | GLU | GLF | LAF | NHF | PRO | ASN |
|---|---|---|---|---|---|---|---|---|
| VCD | 1.00 | | | | | | | |
| GTF | -0.35 | 1.00 | | | | | | |
| GLU | -0.05 | -0.19 | 1.00 | | | | | |
| GLF | 0.05 | -0.12 | 0.38 | 1.00 | | | | |
| LAF | 0.17 | -0.56 | 0.35 | -0.12 | 1.00 | | | |
| NHF | -0.08 | -0.35 | 0.34 | 0.52 | 0.52 | 1.00 | | |
| PRO | 0.46 | -0.20 | 0.20 | 0.53 | 0.31 | 0.59 | 1.00 | |
| ASN | -0.26 | 0.77 | 0.17 | -0.21 | -0.29 | -0.29 | -0.21 | 1.00 |

Table 9: Data correlation analysis map for samples generated with examples 1 and 2 and samples generated by programmed spiking presented in this example. Shaded Grey – autocorrelation (R=1), High correlation (0.8<|R|<1), Medium correlated (0.6<|R|<0.8), Slightly correlated (0.5<|R|<0.6), low or no correlation (0<|R|<0.5).

After sample preparation the spectroscopic sensor or analytical instrument can be used to acquire data (X) that can be related with chemical composition of physical properties of the samples determined by a reference analytical method (y).

Example 4

Example 4 relates to creating data samples to make calibration models more accurate and robust by minimizing unknown or undesired correlations that cannot be measured by analytical methods. Examples of these unknown or undesired correlations are, for example, mixtures of solvents or solutions with different spectra that are not directly correlated with the analyte to be calibrated, dynamic process that change spectra over time without changing the analyte concentration being calibrated, and any other changes that affect the spectra without changing the analyte amount.

When developing calibrations according to example 1 and 2 the samples generated contain highly correlated metabolite concentration as a result of metabolic relations. To overcome this constraints, samples for breaking correlation from example 3 were used. As mentioned before, these samples do no break matrix correlation contained in real fermentation or other dynamic processes. Therefore, an additional dataset was planned based on mixing random amounts of two or more process samples (meaning samples from process experiments, i.e., fermentation runs) chosen in an arbitrary way.

For this example, it will be taken into consideration that a matrix effect is present and that samples from the beginning of the process have different spectra from the remaining process stages. This is the case of a fermentation run, wherein the fermentation feeds do not have the same composition as the fermentation broth and the analytes cannot explain all the variation in the spectra. In these cases the use of an additional dataset containing process samples randomly mixed from different process stages will contribute to reduce the called "matrix effects" not accounted in the analytes profiles, i.e., if samples after feeding present a different spectra due to a different matrix being introduced in the fermentation broth, making random or programed mixtures will help breaking the colinearity due to this effect.

Example 5

Example 5 relates to creating NIRS and MIRS calibrations using samples generated according to the presented method.

The samples generated in Examples 1 trough 4 were used to build multivariate calibrations that relate samples NIR and MIR spectra or parts of that spectrum with a chemical or physical property of a specific sample. The relationship between spectra and chemical and physical properties requires the use of multivariate data analysis methods, such as, Projection into Latent Structures regressions. The optimization of such methods can involve advanced spectra pre-processing, specific wavelength selection, samples selection methods, etc., which are known to skilled persons in both chemometrics and spectroscopies. The details of developing such calibration are not included in this example since they are out of scope of the present invention. As such, the present example serves as a proof that using the samples generated with the presented invention method in accordance with the invention, it is possible to create calibrations that can be used in real fermentation runs.

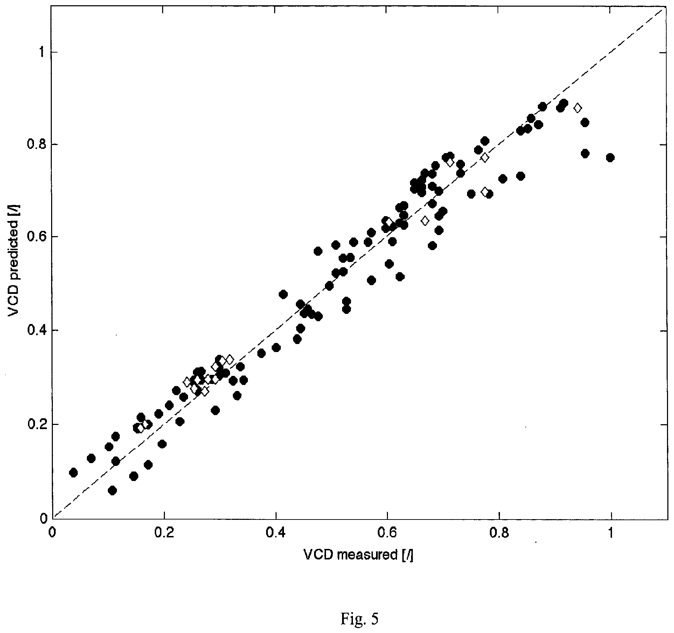

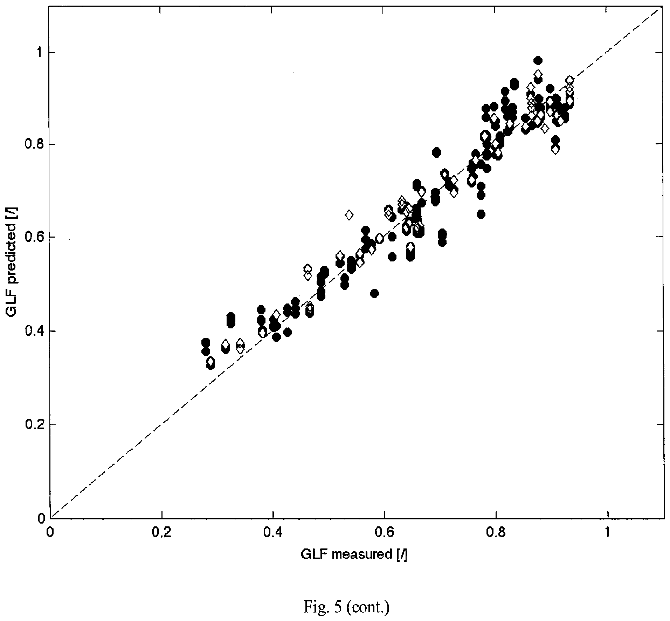

For that, the spectra (NIR, MIR, or other not presented in this example) of all samples generated in Examples 1, 2, 3 and 4 were acquired and the analytical properties for such samples were obtained either by laboratory analytical methods or estimated from main solutions. The calibrations for the analytes presented in Examples 1, 2, 3 and 4 were developed using good modelling practices, which are known to people skilled in this area. The model performance for the determination of viable cell density with NIRS and glucose using MIRS are presented here as an example of possible applications of the present invention (Fig. 5A and 5B, respectively), but do not limit the extent of this invention to other properties, other processes and other analytical techniques.

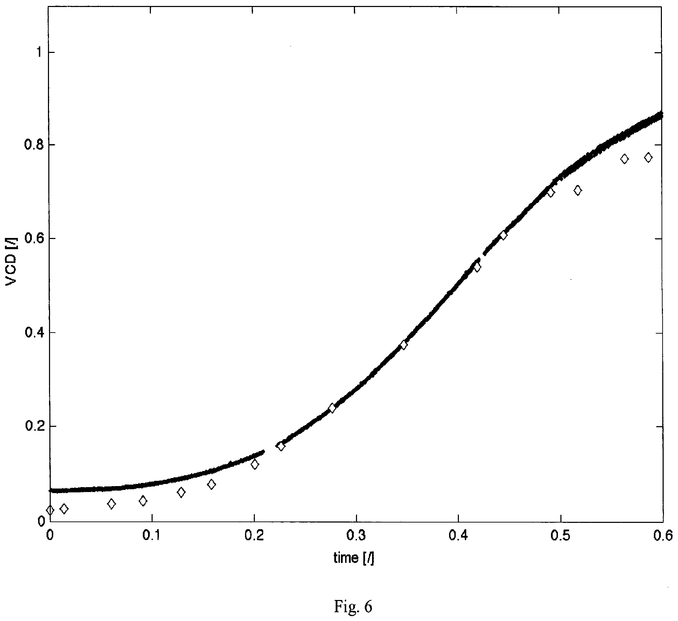

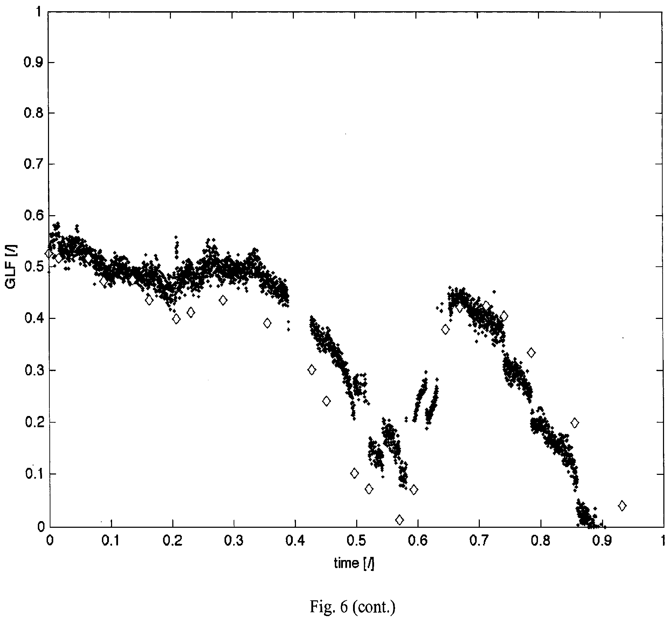

The models presented above were challenged by using them to predict viable cell density and glucose in real fermentation runs. The results presented in Fig. 6A and 6B, clearly show the successful use of the presented method in using synthetic multivariate and programmed spiking samples, acquired at a 100 mL scale, to develop calibrations for on-line monitoring of real fermentation runs. It is worth mention that the fermentation run was conducted in a 10 L fermenter (100:1 scale up). Even so, the predicted viable cell density and glucose profiles for the on-line monitoring of fermentations are in great accordance with the reference analytics measured by reference methods.

Regarding the results obtained for VCD model two other things have to be considered. First, the model built with synthetic multicomponent samples does not have information about morphology/cell state along the simulated fermentation, i.e., cells introduced in the engineered samples are not at the same state as if they were in a real fermentation. This clearly influences model performance, especially at the end of the fermentation run, where the number of cells is high but the viable cells start to decrease.

Example 6

Example 6 relates to multivariate process trajectories development using synthetic multicomponent samples.

Spectroscopic techniques, such as those presented in Example 5 can be very valuable tools to monitor fermentation runs. Also, the use of these spectroscopic techniques is not limited to the development of calibrations for monitoring/control single process events. In fact, monitoring process trajectories is an interesting area where chemometric tools can play an important role. The great advantage of these tools is the ability to capture much more than the individual analytes profiles, and as such, they can be very useful for process supervision and control, even without quantification methods.

The presented method according to the invention also allows the creation of multivariate process trajectories based on the samples created in Examples 1 and 2, since these samples present the same characteristics as real fermentation samples. As such, using spectroscopic sensors information it is possible to build multivariate batch trajectories that can be used to benchmark current fermentation runs with prior batches and identify deviations.

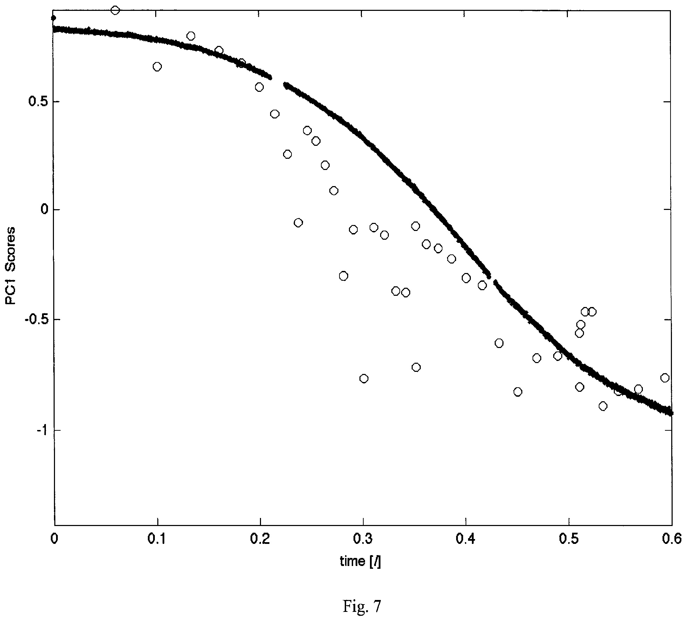

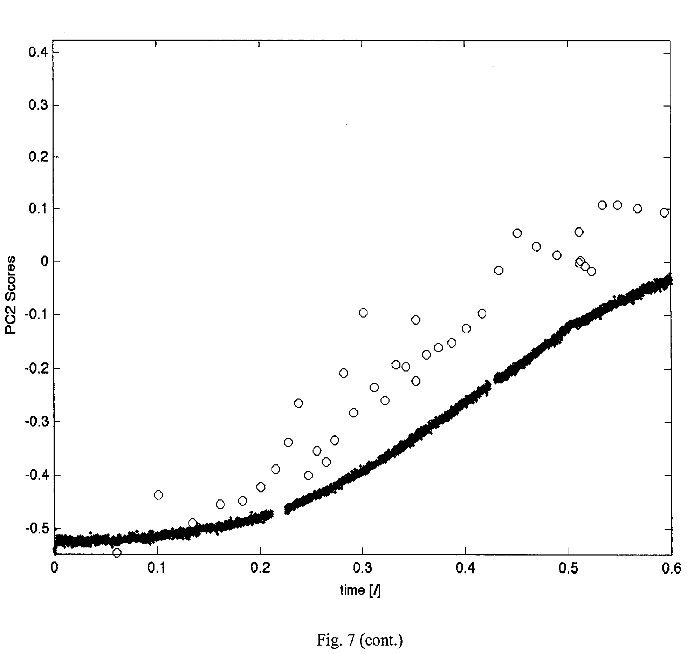

To exemplify the use of this technique the samples produced in Example 1 and 2 were used to build a NIRS PCA model according to good modelling practices. After model optimization, the spectra from a fermentation run were acquired inline. These spectra were projected on the PCA model to monitor the batch performance. As it can be seen in Fig. 7A and 7B, the batch multivariate trend produced by the online measurements of the fermentation run presents the same profile as the synthetic multicomponent samples. Although the trends are captured, it is possible to notice some deviations starting around 70 to 90 h (especially on Fig. 7B). It is worth mention that the deviations were caused by a time shift in the feeding streams; otherwise, the profiles would be perfectly aligned. As such, the synthetic multicomponent samples can be used to produce multivariate profiles to monitor fermentations performances. Furthermore, the created batch profiles can be used in MSPC control charts to better visualize the deviations and to diagnose their causes.

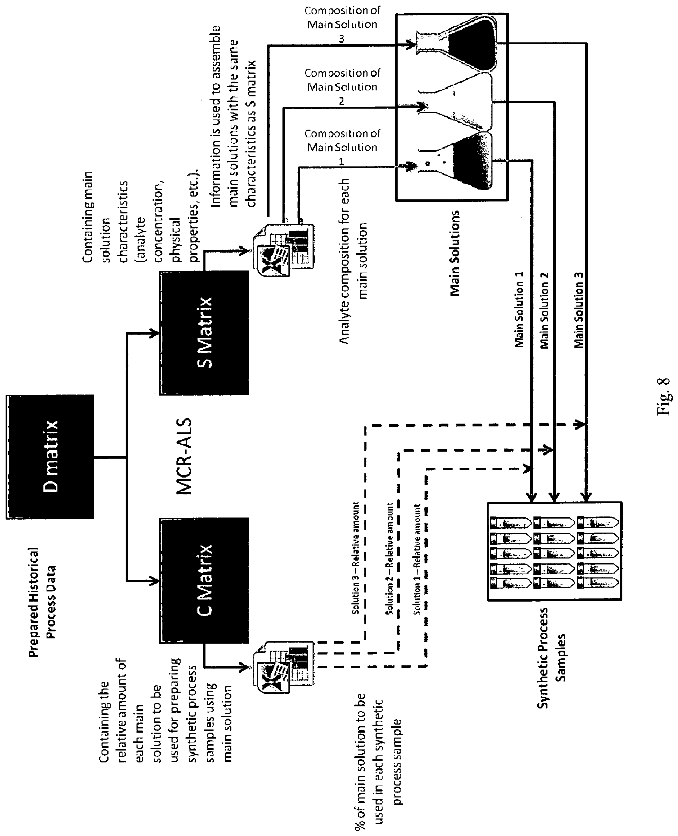

Figure 8 shows a diagram according to an embodiment of the invention. In a first step the historical process data D of the dynamic process which has to be mimicked is created and prepared. In a second step Multivariate curve resolution - alternating least squares algorithm (MCR-ALS) is performed. Using this algorithm, it is determined which main solutions of the data matrix D are necessary to mimic the dynamic process within a predetermined variance. Then, the analyte composition of the necessary main solutions and the respective relative amount of the necessary main solutions with respect to the data matrix D are determined.

The analyte composition as well as optional other solution characteristics, as e.g. physical properties etc., of the necessary main solutions are contained in a Matrix S and the relative amount of each main solution to be used for preparing/creating the synthetic process samples are contained in a matrix C.

With the information in the matrix S, which main solutions are necessary to create the at least one sample mimicking the dynamic process and which analyte composition these necessary main solution have, the necessary main solutions are assembled/mixed according to the determined analyte composition.

Then, with the information in the matrix C and which relative amount the assembled necessary main solutions have, the assembled necessary main solutions are mixed according to their determined respective relative amount. With this the at least one sample mimicking the dynamic process is created.

It is understood by the skilled person that according to another embodiment the analyte composition as well as optional other solution characteristics, as e.g. physical properties etc., of the necessary main solutions do not have to be necessarily contained in a Matrix S but can also be contained in any other representation, e.g. an ordinary table. According to another embodiment the relative amount of each main solution which is used for preparing/creating the synthetic process samples does also not necessarily have to be contained in a matrix C but can also be contained in any other representation, e.g. an ordinary table.

While the invention has been illustrated and described in detail in the drawings and foregoing description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below.

The invention also covers all further features shown in the Figs. individually although they may not have been described in the afore or following description. Also, single alternatives of the embodiments described in the figures and the description and single alternatives of features thereof can be disclaimed from the subject matter of the invention.

Furthermore, in the claims the word "comprising" does not exclude other elements or steps, and the indefinite article "a" or "an" does not exclude a plurality. A single unit or step may fulfil the functions of several features recited in the claims. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. A computer program may be stored/distributed on a suitable medium, such as an optical storage medium or a solid-state medium supplied together with or as part of other hardware, but may also be distributed in other forms, such as via the Internet or other wired or wireless telecommunication systems. In particular, e.g., a computer program can be a computer program product stored on a computer readable medium which computer program product can have computer executable program code adapted to be executed to implement a specific method such as the method according to the invention. Any reference signs in the claims should not be construed as limiting the scope.

Terminology and abbreviations

In the context of the invention, the terms and abbreviations listed below can related to the following mentioned for the respective terms and abbreviations:

Accurate calibration: The calibration can more or less correctly predict the amount of a specific analyte, i.e., with a comparably low deviation from actual value.

ASN: Asparagine.

C: In the present disclosure, C represents the matrix containing the relative amount of each main solution obtained from the MCR-ALS results.

$C_{aug}$: C matrix augmented, e.g. according to Example 2 above.

Chemometric methods: Application of mathematical or statistical methods to relate measurements made on a chemical system or process (for example, pressure, flow, temperature, pH, near & mid infrared spectra, 2d fluorescence spectra, dielectric spectra, Raman spectra, NMR spectra, etc) to the state of the systems (physical or chemical properties).

Closure Constraint: This constraint state that the MCR-ALS solution for C matrix have to provide relative amount, i.e., the sum of the row of C has to be equal to one.

Co-linearity breaking samples: Samples that are not related to the process trends.

CQAs: Critical quality atributes.

D: Data matrix containing the historical data of a batch. D can be a n x m matrix that has n rows (samples) and m columns (properties).

$D_i$: Part of original data matrix D.

Dynamic Processes: Continuous, batch or fed-batch process that changes or progresses along time. In the sense of the present disclosure it can be interpreted as a reaction (fermentation or chemical), a mixture, a dissolution, evaporation, drying, etc. In continuous operation, dynamic process is considered to be as an operational conditions change, i.e., a disturbance or a programmed change is the variables that have impact on the product quality specifications.

GLF: Glucose.

GLU: Glutamate.

GTF: Glutamine.

Historical Data: Registered past information about a process, that can be composed by process variables and also chemical/physical information at different times of the process run. This data can be used to develop multivariate calibration models, or to supervise the current batch when compared with prior batches.

In-line: That is measured inside the process equipment.

In-process samples: Samples from the process as opposed to raw material and final product.

LAF: Lactate.

LV: Latent variables used in PLS regressions.

MCR-ALS: Multivariate curve resolution - alternating least squares method or algorithm.

Mimic: It is used in a sense that it has similar or the same characteristics as a real fermentation, i.e., that follows the same profile as a real fermentation. Relating to samples means that they have the same profiles.

MIRS: MID infrared spectroscopy

MSPC: Multivariate statistical process control. Chemometric tool that uses prior historical data or sensors data from good batches and compares it with a current batch to detect deviations and find their root causes.

Multicomponent Analyte Composition: Chemical composition of a sample comprised of 2 or more analytes.

Multi-parameter analysis: This expression is used in a context that one measurement using spectroscopic sensor can be used to predict several properties of a given sample.

Multivariate Calibrations: Models that can be used to predict a specific property measurement, such as, chemical composition or physical property or other, based on a measured process parameter (for example, pressure, flow, temperature, pH, etc.) or instrument signal (for example, near & mid infrared, 2d fluorescence, dielectric spectroscopy, Raman, NMR spectra, etc.)

NHF: Ammonia.

NIRS: Near infrared spectroscopy.

Non-negativity constraint: This constraint states that MCR-ALS solutions have to be positive.

PCA: Principal component analysis.

PLS: Projection into Latent Structures.

PLS-DA: Projection into Latent Structures discriminant Analysis, a chemometric method used to discriminate samples based on a specific attribute. In this method the samples that have that attribute are coded with number 1 and the remaining are coded as 0. A PLS model is developed to discriminate both groups. When a new unknown sample is presented to the calibration model a prediction is made and the closer to 1 the higher the probability is that this sample has this attribute.

PRO: Product.

Process trajectories: Multivariate trend of a batch generally captured by a chemometric method such as PCA.

Programmed spiking: Guided spiking to reach desired sample characteristics.

R: Correlation coefficient.

Random spiking: Computer generated spiking with no specific pattern.

RMSEC: Root mean square error of calibration which can be calculated from the distance of the predicted value for calibration samples to their actual value.

RMSECV: Root mean square error of cross validation. Used for model optimization by developing several calibrations while setting aside a group of samples. This is an iterative process and the samples removed in the first step are used for calibration while another set of samples is removed. This procedure is repeated several times and allows for optimization of the number of latent variables.

RMSEP: Root mean square error of prediction for the external validation samples. Used to measure the model prediction capability to samples that are not included in the calibration.

Robust calibrations: Meaning that the calibrations can still predict accurately even if the sample has some interferences (physical or chemical).

SEL: Standard error of laboratory which can be an error of the analytical method used to determine a specific property of the samples.

SIMCA: Soft independent class analysis, a chemometric method to discriminate samples based on clustering and distance between samples.

SIMPLISMA: Algorithm called Simple-to-use Interactive Self-modeling Mixture Analysis that can be used for extracting pure component spectra from a series of spectroscopic observations of a mixture while the component concentrations varies.

$Spk_i$: Spike number i.

$S^T$: In the present disclosure, $S^T$ is the matrix containing the multicomponent multicomponent analyte composition of each main solution obtained from the MCR-ALS results when using matrix D.

$S^T_i$: In the present disclosure, $S^T_i$ is the matrix containing the multicomponent analyte composition of each main solution obtained from the MCR-ALS results when using matrix $D_i$.

Supervision System: System that compares previous batches (dynamic processes) profiles/trends with the current batch profile/trend. This system should have an interface so the operator clearly identifies deviations from previous batches, and take the proper measures by control actions, either manually or automatically. This system can be done including both process variables trends and spectroscopy sensors multivariate trajectories obtained by a chemometric method such as PCA or PLS. The root cause effects can be also available in the interface.

Synthetic Multicomponent Samples: Samples created in a laboratory or in controlled engineering conditions as opposed to samples withdrawn from a process. The term multicomponent means that the sample is composed by several chemical compounds and also can include suspended live materials, such as cells.

t: Time.

VCD: Viable cell density.

X: Sensor data, for instance NIR and MIR spectra, etc.

y: Property to be modelled that has to be determined by a reference method, for instance, VCD, GLF, GLU, GTF, etc. concentration.

a: Standard deviation. The mean standard deviation is used when a standard error of laboratory is not available.

Bibliography

[1] de Juan, Anna, and Roma Tauler. "Chemometrics applied to unravel multicomponent processes and mixtures. Revisiting latest trends in multivariate resolution." Analytica Chimica Acta 500 (2003): 195-210.

[2] Gampp, H, M Maeder, C J Meyer, and A D Zuberbuhler. "Calculation of equilibrium constants from multiwavelength spectroscopic data-IV: Model-free least squares refinement by use of evolving factor analysis." Talanta 33 (1986): 943-951.

[3] Jaumout, Joaquim, Raimundo Gargallo, Anna de Juan, and Roma Tauler. "A graphical user-friendly interface for MCR-ALS: a new tool for multivariate curve resolution in MATLAB." Chemometrics and Intelligent Laboratory Systems 76 (2005): 101-110.

[4] Windig, W, and D A Stephenson. "Self-modeling mixture analysis of second-derivative near infrared spectral data using the SIMPLISMA approuch." Analytical Chemistry 64 (1992): 2735-2742.

**Claims**

1. A method for preparing at least one synthetic multicomponent biotechnological and/or chemical process sample for developing multivariate calibrations for monitoring systems and/or multivariate supervisory systems of increased robustness, wherein at least one synthetic sample mimicking a dynamic process or a specific process step or a variation thereof is prepared, the method comprising the steps of:

preparing historical process data of the dynamic process or the specific process step or the variation thereof to be mimicked and for which the at least one synthetic sample is to be prepared;

creating a data matrix D of the historical process data;

determining a plurality of main solutions of the data matrix D;

determining which main solutions of the data matrix D are necessary to mimic the dynamic process or the specific process step or the variation thereof within a predetermined variance;

determining the analyte composition of the necessary main solutions and the respective relative amount of the necessary main solutions with respect to the data matrix D;

creating a matrix S comprising the determined analyte composition of the necessary main solutions;

creating a matrix C comprising the relative amount of the necessary main solutions with respect to the data matrix D;

determining whether the matrix product C x ST corresponds to the data matrix D within a predetermined tolerance;

determining the relative amount of the necessary main solutions comprised in the matrix C for at least two instants of time;

performing a regression between the relative amount of the necessary main solutions comprised in the matrix C and a respective time variable of the historical process data using the determined relative amount of the necessary main solutions for the at least two instants of time;

estimating the relative amount of the necessary main solutions for at least one other instant of time between the at least two instants of time based on the regression; and

creating an augmented time dependent matrix $C_{aug}$ comprising C and the estimated necessary main solutions for the at least one other instant of time,

wherein the aforementioned steps are carried out on a computational unit; and

wherein the method further comprises the step of:

creating at least one sample by assembling the necessary main solutions according to the determined analyte composition.

**2.** The method according to claim 1, wherein if the matrix product C x $S^T$ does not correspond to the data matrix D within the predetermined tolerance repeating the steps of:

> determining which main solutions of the data matrix D are necessary to mimic the dynamic process or the specific process step or the variation thereof within the predetermined variance;
> determining the analyte composition of the necessary main solutions and the relative amount of each necessary main solution;
> creating the matrix S;
> creating the matrix C;
> determining whether the matrix product C x $S^T$ corresponds to the data matrix D within the predetermined tolerance.

**3.** The method according to claim 1 or 2, wherein the relative amount of the necessary main solutions for creating the data matrix C and/or the determined analyte composition of the necessary main solutions for creating the matrix S is selected based on logical constraints and/or physical constraints and/or chemical constraints.

**4.** The method according to any of claims 1 to 3,
wherein the data matrix D is partitioned into a plurality of data matrices Di and
wherein each matrix Di is individually processed in each step following the creating of the data matrix D, preferably if it is determined that only main solutions of the data matrix D can be determined which are configured to mimic the dynamic process or the specific process step or the variation thereof below the predetermined variance.

**5.** The method according to any of claims 1 to 4, wherein the historical data comprises at least one of the following: analytically determined composition values of the dynamic process or the specific process step or the variation thereof, physical information, process information, data of at least one further process variation corresponding to additional experimental and/or simulated runs of the process under similar or different process conditions and/or wherein the historical data is organized in the matrix D according to the process time.

**6.** The method according to any of claims 1 to 5, wherein determining the necessary main solutions and/or determining the analyte composition of the necessary main solutions and/or determining the relative amount of the necessary main solutions with respect to the data matrix D is performed using an appropriate chemometric method, in particular at least one of the following chemometric methods:

> a factor decomposition method, in particular principal component analysis (PCA), singular value decomposition (SVD) or evolving factor analysis,
> and/or
> a parallel factor analysis (PARAFAC), multivariate curve resolution-alternating least squares (MCR-ALS), evolving factor analysis.

**7.** The method according to any of claims 1 to 6, further comprising:
preprocessing the matrix D, preferably filtering, more preferably using at least one of: a Savitzky-Golay filter, a Kernel smoother, a smoothing spline, a moving average, or a weighted moving average.

**8.** The method according to any of claims 1 to 7, further comprising:
preparing at least one second sample being configured to break co-linearity between parameters or analytes comprised in the data matrix D using a co-linearity breaking method.

**9.** The method according to claim 8, wherein the co-linearity breaking method comprises at least one of the following: creating of an orthogonal design of experiments, random spiking of the specific compounds in the at least one sample, programmed spiking, random mixing of samples or any combination thereof.

**10.** The method according to claim 8 or 9, further comprising:
creating a third sample by grouping the sample with the at least one second sample.

**11.** A synthetic sample layout generation and sample handling system for preparing at least one synthetic sample mimicking a dynamic process or a specific process step or a variation thereof, the system being adapted to carry out the method according to any one of the preceding claims and the system comprising:

a computational unit; and
a mixing unit;
wherein the computational unit comprises:

means for preparing historical process data of the dynamic process or the specific process step or the variation thereof to be mimicked and for which the at least one synthetic sample is to be prepared;
means for creating a data matrix D of the historical process data;
means for determining a plurality of main solutions of the data matrix D;
means for determining which main solutions of the data matrix D are necessary to mimic the dynamic process or the specific process step or the variation thereof within a predetermined variance;
means for determining the analyte composition of the necessary main solutions and the respective relative amount of the necessary main solutions with respect to the data matrix D; and
wherein the mixing unit is configured to create the at least one sample by assembling the necessary main solutions according to the determined analyte composition,

wherein the computational unit further comprises:

means for creating a matrix S comprising the determined analyte composition of the necessary main solutions;
means for creating a matrix C comprising the relative amount of the necessary main solutions with respect to the data matrix D;
means for determining whether the matrix product C x $S^T$ corresponds to the data matrix D within a predetermined tolerance;
wherein the means for determining the respective relative amount of the necessary main solutions with respect to the data matrix D are configured to determine the relative amount of the necessary main solutions comprised in the matrix C for at least two instants of time; the computational unit further comprising:

means for performing a regression between the relative amount of the necessary main solutions comprised in the matrix C and a respective time variable of the historical process data using the determined relative amount of the necessary main solutions for the at least two instants of time;
means for estimating the relative amount of the necessary main solutions for at least one other instant of time between the at least two instants of time based on the regression; and
means for creating an augmented time dependent matrix $C_{aug}$ comprising C and the estimated necessary main solutions for the at least one other instant of time.

12. The system according to claim 11, further comprising a measurement device having at least one sensor.

13. The system according to claim 12, wherein the at least one sensor of the measurement device is configured to measure at least one parameter in-situ in the dynamic process, the specific process step or the variation thereof and/or wherein the at least one sensor comprises at least one of the following: a pH probe, a temperature probe, a spectroscopic sensor, or a multichannel instrument.

14. The system according to any of claims 11 to 13, further comprising at least one control device comprising at least one temperature control structure and/or at least one pH control structure.

15. The system according to claim 14, wherein the at least one control device is configured to recreate respective conditions of the dynamic process or the specific process step or the variation thereof.

16. The system according to claim 13 or 14, wherein the at least one control device is configured to break the co-linearity between parameters or analytes comprised in the data matrix D using a co-linearity breaking method.

17. The system according to claim 16, wherein the at least one control device is configured to break the co-linearity using at least one of the following co-linearity breaking methods: creating of an orthogonal design of experiments, random spiking of the specific compounds in the at least one sample, programmed spiking, random mixing of samples or any combination thereof.

18. The system according to any of claims 11 to 17 further comprising an analytical device configured to analyze at least one of the main solutions and/or the at least one sample.

19. A computer program product comprising one or more computer readable media having computer executable instructions for performing the steps of the method of any of claims 1 to 10.

**Patentansprüche**

1. Verfahren zur Herstellung wenigstens einer synthetischen biotechnologischen Mehrkomponenten- und/oder chemischen Prozessprobe zur Entwicklung multivariater Kalibrierungen für Überwachungssysteme und/oder multivariater Aufsichtssysteme mit erhöhter Robustheit, wobei wenigstens eine synthetische Probe einen dynamischen Prozess oder einen spezifischen Prozessschritt imitiert oder eine Variation davon hergestellt wird, wobei das Verfahren die folgenden Schritte aufweist:

   Erzeugen historischer Prozessdaten des dynamischen Prozesses oder des spezifischen Prozessschritts oder der Variation davon, der/die imitiert werden soll und für den/die die wenigstens eine synthetische Probe hergestellt werden soll;
   Erzeugen einer Datenmatrix D der historischen Prozessdaten;
   Bestimmen einer Vielzahl von Hauptlösungen der Datenmatrix D;
   Bestimmen, welche Hauptlösungen der Datenmatrix D notwendig sind, um den dynamischen Prozess oder den spezifischen Prozessschritt oder deren Variation innerhalb einer vorgegebenen Abweichung zu imitieren;
   Bestimmen der Analytzusammensetzung der notwendigen Hauptlösungen und der jeweiligen relativen Menge der notwendigen Hauptlösungen in Bezug auf die Datenmatrix D;
   Erzeugen einer Matrix S, die die bestimmte Analytzusammensetzung der notwendigen Hauptlösungen aufweist;
   Erzeugen einer Matrix C, die die relative Menge der notwendigen Hauptlösungen in Bezug auf die Datenmatrix D aufweist;
   Bestimmen, ob das Matrixprodukt C x ST innerhalb einer vorgegebenen Toleranz der Datenmatrix D entspricht;
   Bestimmen der relativen Menge der notwendigen Hauptlösungen, die in der Matrix C enthalten sind, für wenigstens zwei Zeitpunkte;
   Durchführen einer Regression zwischen der relativen Menge der notwendigen Hauptlösungen, die in der Matrix C enthalten sind, und einer jeweiligen Zeitvariablen der historischen Prozessdaten unter Verwendung der bestimmten relativen Menge der notwendigen Hauptlösungen für die wenigstens zwei Zeitpunkte;
   Schätzen der relativen Menge der notwendigen Hauptlösungen für den wenigstens einen anderen Zeitpunkt zwischen den wenigstens zwei Zeitpunkten basierend auf der Regression; und
   Erzeugen einer erweiterten zeitabhängigen Matrix $C_{aug}$, die C und die geschätzten notwendigen Hauptlösungen für den wenigstens einen anderen Zeitpunkt aufweist, wobei die vorstehend erwähnten Schritte auf einer Recheneinheit ausgeführt werden; und wobei das Verfahren ferner den folgenden Schritt aufweist:
   Erzeugen wenigstens einer Probe durch Zusammensetzen der notwenigen Hauptlösungen gemäß der bestimmten Analytzusammensetzung.

2. Verfahren nach Anspruch 1, wobei, wenn das Matrixprodukt $C \times S^T$ nicht innerhalb der vorgegebenen Toleranz der Datenmatrix D entspricht, Wiederholen der folgenden Schritte:

   Bestimmen, welche Hauptlösungen der Datenmatrix D notwendig sind, um den dynamischen Prozess oder den spezifischen Prozessschritt oder deren Variation innerhalb der vorgegebenen Abweichung zu imitieren;
   Bestimmen der Analytzusammensetzung der notwendigen Hauptlösungen und der relativen Menge jeder notwendigen Hauptlösung;
   Erzeugen der Matrix S;
   Erzeugen der Matrix C;
   Bestimmen, ob das Matrixprodukt $C \times S^T$ innerhalb der vorgegebenen Toleranz der Datenmatrix D entspricht.

3. Verfahren nach Anspruch 1 oder 2, wobei die relative Menge der notwendigen Hauptlösungen zur Erzeugung der Datenmatrix C und/oder der bestimmten Analytzusammensetzung der notwendigen Hauptlösungen zur Erzeugung der Matrix S basierend auf logischen Nebenbedingungen und/oder physikalischen Nebenbedingungen und/oder chemischen Nebenbedingungen ausgewählt wird.

4. Verfahren nach einem der Ansprüche 1 bis 3,
   wobei die Datenmatrix D in mehrere Datenmatrizen Di zerlegt wird, und wobei vorzugsweise jede Matrix Di in jedem Schritt anschließend an die Erzeugung der Datenmatrix D einzeln verarbeitet wird, wenn bestimmt wird, dass nur Hauptlösungen der Datenmatrix D bestimmt werden können, die konfiguriert sind, um den dynamischen Prozess oder den spezifischen Prozessschritt oder deren Variation unterhalb der vorgegebenen Abweichung zu imitieren.

5. Verfahren nach einem der Ansprüche 1 bis 4, wobei die historischen Daten wenigstens eines der folgenden aufweisen: analytisch bestimmte Zusammensetzungswerte des dynamischen Prozesses oder des spezifischen Prozessschritts oder deren Variation, physikalische Informationen, Prozessinformationen, Daten wenigstens einer weiteren Prozessvariation, die zusätzlichen experimentellen und/oder simulierten Läufen des Prozesses unter ähnlichen oder unterschiedlichen Prozessbedingungen entspricht, und/oder wobei die historischen Daten in der Matrix D gemäß der Prozesszeit organisiert sind.

6. Verfahren nach einem der Ansprüche 1 bis 5, wobei das Bestimmen der notwendigen Hauptlösungen und/oder das Bestimmen der Analytzusammensetzung der notwendigen Hauptlösungen und/oder das Bestimmen der relativen Menge der notwendigen Hauptlösungen in Bezug auf die Datenmatrix D unter Verwendung eines geeigneten chemometrischen Verfahrens, insbesondere wenigstens eines der folgenden chemometrischen Verfahren, durchgeführt wird:

ein Faktorzerlegungsverfahren, insbesondere Hauptkomponentenanalyse (PCA), Singulärwertzerlegung (SVD) oder entwickelnde Faktoranalyse,
und/oder
eine Parallelfaktoranalyse (PRAFAC), multivariate Kurvenauflösung mit abwechselnden kleinsten Quadraten (MCR-ALS), entwickelnde Faktoranalyse.

7. Verfahren nach einem der Ansprüche 1 bis 6, das ferner aufweist:
Vorverarbeiten der Matrix D, vorzugsweise Filtern, noch besser unter Verwendung wenigstens eines der Folgenden: ein Savitzky-Golay-Filter, einen Kernel-Glätter, eine Spline-Glättung, ein gleitendes Mittel oder ein gewichtetes gleitendes Mittel.

8. Verfahren nach einem der Ansprüche 1 bis 7, das ferner aufweist:
Herstellen wenigstens einer zweiten Probe, die konfiguriert ist, um die Kollinearität zwischen Parametern oder Analyten, die in der Datenmatrix D enthalten sind, unter Verwendung eines Kollinearitätsaufspaltungsverfahrens aufzuspalten.

9. Verfahren nach Anspruch 8, wobei das Kollinearitätsaufspaltungsverfahren wenigstens eines der folgenden aufweist: Erzeugen eines orthogonalen Entwurfs von Experimenten, zufälliges Aufstocken der spezifischen Verbindungen in der wenigstens einen Probe, programmiertes Aufstocken, zufälliges Mischen von Proben oder jede Kombination davon.

10. Verfahren nach Anspruch 8 oder 9, das ferner aufweist:
Erzeugen einer dritten Probe durch Gruppieren der Probe mit der wenigstens einen zweiten Probe.

11. System zur Erzeugung einer synthetischen Probenanordnung und zur Probenhandhabung zur Herstellung wenigstens einer synthetischen Probe, die einen dynamischen Prozess oder einen spezifischen Prozessschritt oder eine Variation davon imitiert, wobei das System geeignet ist, das Verfahren nach einem der vorhergehenden Ansprüche auszuführen, und wobei das System aufweist:

eine Recheneinheit; und
eine Mischeinheit;
wobei die Recheneinheit aufweist:

Einrichtungen zum Erzeugen historischer Prozessdaten des dynamischen Prozesses oder des spezifischen Prozessschritts oder der Variation davon, der/die imitiert werden soll und für den/die die wenigstens eine synthetische Probe hergestellt werden soll;
Einrichtungen zum Erzeugen einer Datenmatrix D der historischen Prozessdaten;
Einrichtungen zum Bestimmen einer Vielzahl von Hauptlösungen der Datenmatrix D;
Einrichtungen zum Bestimmen, welche Hauptlösungen der Datenmatrix D notwendig sind, um den dynamischen Prozess oder den spezifischen Prozessschritt oder deren Variation innerhalb einer vorgegebenen Abweichung zu imitieren;
Einrichtungen zum Bestimmen der Analytzusammensetzung der notwendigen Hauptlösungen und der jeweiligen relativen Menge der notwendigen Hauptlösungen in Bezug auf die Datenmatrix D; und

wobei die Mischeinheit konfiguriert ist, um die wenigstens eine Probe durch Zusammensetzen der notwendigen Hauptlösungen gemäß der bestimmten Analytzusammensetzung zu erzeugen,

wobei die Recheneinheit ferner aufweist:

Einrichtungen zum Erzeugen einer Matrix S, die die bestimmte Analytzusammensetzung der notwendigen Hauptlösungen aufweist;
Einrichtungen zum Erzeugen einer Matrix C, die die relative Menge der notwendigen Hauptlösungen in Bezug auf die Datenmatrix D aufweist;
Einrichtungen zum Bestimmen, ob das Matrixprodukt $C \times S^T$ innerhalb einer vorgegebenen Toleranz der Datenmatrix D entspricht;
wobei die Einrichtungen zum Bestimmen der jeweiligen relativen Menge der notwendigen Hauptlösungen in Bezug auf die Datenmatrix D konfiguriert sind, um die relative Menge der notwendigen Hauptlösungen, die in der Matrix C enthalten sind, für wenigstens zwei Zeitpunkte zu bestimmen; wobei die Recheneinheit ferner aufweist:

Einrichtungen zum Durchführen einer Regression zwischen der relativen Menge der notwendigen Hauptlösungen, die in der Matrix C enthalten sind, und einer jeweiligen Zeitvariablen der historischen Prozessdaten unter Verwendung der bestimmten relativen Menge der notwendigen Hauptlösungen für die wenigstens zwei Zeitpunkte;
Einrichtungen zum Schätzen der relativen Menge der notwendigen Hauptlösungen für den wenigstens einen anderen Zeitpunkt zwischen den wenigstens zwei Zeitpunkten basierend auf der Regression; und
Einrichtungen zum Erzeugen einer erweiterten zeitabhängigen Matrix $C_{aug}$, die C und die geschätzten notwendigen Hauptlösungen für den wenigstens einen anderen Zeitpunkt aufweist.

12. System nach Anspruch 11, das ferner eine Messvorrichtung mit wenigstens einem Sensor aufweist.

13. System nach Anspruch 12, wobei der wenigstens eine Sensor der Messvorrichtung konfiguriert ist, um wenigstens einen Parameter an Ort und Stelle in dem dynamischen Prozess, dem spezifischen Prozessschritt oder deren Variation zu messen, und/oder wobei der wenigstens eine Sensor wenigstens einen der folgenden aufweist; eine pH-Sonde, eine Temperatursonde, einen spektroskopischen Sensor oder ein Mehrkanalmessinstrument.

14. System nach einem der Ansprüche 11 bis 13, das ferner wenigstens eine Steuervorrichtung aufweist, die wenigstens eine Temperatursteuerstruktur oder wenigstens eine pH-Steuerstruktur aufweist.

15. System nach Anspruch 14, wobei die wenigstens eine Steuervorrichtung konfiguriert ist, um jeweilige Bedingungen des dynamischen Prozesses oder des spezifischen Prozessschritts oder deren Variation nachzubilden.

16. System nach Anspruch 13 oder 14, wobei die wenigstens eine Steuervorrichtung konfiguriert ist, um die Kollinearität zwischen Parametern oder Analyten, die in der Datenmatrix D enthalten sind, unter Verwendung eines Kollinearitätsaufspaltungsverfahrens aufzuspalten.

17. System nach Anspruch 16, wobei die wenigstens eine Steuervorrichtung konfiguriert ist, um die Kollinearität unter Verwendung wenigstens eines der folgenden Kollinearitätsaufspaltungsverfahren aufzuspalten: Erzeugen eines orthogonalen Entwurfs von Experimenten, zufälliges Aufstocken der spezifischen Verbindungen in der wenigstens einen Probe, programmiertes Aufstocken, zufälliges Mischen von Proben oder jede Kombination davon.

18. System nach einem der Ansprüche 11 bis 17, das ferner eine analytische Vorrichtung aufweist, die konfiguriert ist, um wenigstens eine der Hauptlösungen und/oder die wenigstens eine Probe zu analysieren.

19. Computerprogrammprodukt, das ein oder mehrere computerlesbare Medien mit computerausführbaren Anweisungen aufweist, um die Schritte des Verfahrens nach einem der Ansprüche 1 bis 10 durchzuführen.

**Revendications**

1. Procédé de préparation d'au moins un échantillon synthétique et à composants multiples de procédés biotechnologiques et/ou chimiques pour développer des étalonnages à variables multiples pour des systèmes de surveillance et/ou des systèmes de supervision à variables multiples de robustesse accrue, dans lequel au moins un échantillon synthétique simulant un procédé dynamique ou une étape de procédé spécifique ou une variante de celle-ci est préparé, le procédé comprenant les étapes de :

préparation de données historiques de procédé du procédé dynamique ou de l'étape de procédé spécifique ou de la variante de celle-ci à simuler et pour lequel/laquelle l'au moins un échantillon synthétique doit être préparé ;
création d'une matrice de données D des données historiques de procédé ;
détermination d'une pluralité de solutions principales de la matrice de données D ;
détermination des solutions principales de la matrice de données D qui sont nécessaires pour simuler le procédé dynamique ou l'étape de procédé spécifique ou la variante de celle-ci dans les limites d'une variance prédéterminée ;
détermination de la composition des analytes des solutions principales nécessaires et de la quantité relative respective des solutions principales nécessaires par rapport à la matrice de données D ;
création d'une matrice S comprenant la composition déterminée des analytes des solutions principales nécessaires ;
création d'une matrice C comprenant la quantité relative des solutions principales nécessaires par rapport à la matrice de données D ;
détermination si le produit de la matrice C x ST correspond à la matrice de données D dans les limites d'une tolérance prédéterminée ;
détermination de la quantité relative des solutions principales nécessaires comprise dans la matrice C pour au moins deux instants de temps ;
réalisation d'une régression entre la quantité relative des solutions principales nécessaires comprise dans la matrice C et une variable de temps respective des données historiques de procédé en utilisant la quantité relative déterminée des solutions principales nécessaires pour les au moins deux instants de temps ;
estimation de la quantité relative des solutions principales nécessaires pour au moins un autre instant de temps entre les au moins deux instants de temps sur la base de la régression ; et
création d'une matrice augmentée $C_{aug}$ dépendante du temps, comprenant C et les solutions principales nécessaires estimées pour l'au moins un autre instant de temps, dans lequel les étapes mentionnées ci-dessus sont réalisées sur une unité de calcul ; et
dans lequel le procédé comprend en outre l'étape de :
création d'au moins un échantillon par assemblage des solutions principales nécessaires selon la composition déterminée des analytes.

**2.** Procédé selon la revendication 1, dans lequel si le produit de la matrice C x $S^T$ ne correspond pas à la matrice de données D dans les limites de la tolérance prédéterminée, répétition des étapes de :

détermination des solutions principales de la matrice de données D qui sont nécessaires pour simuler le procédé dynamique ou l'étape de procédé spécifique ou la variante de celle-ci dans les limites de la variance prédéterminée ;
détermination de la composition des analytes des solutions principales nécessaires et de la quantité relative de chaque solution principale nécessaire ;
création de la matrice S ;
création de la matrice C ;
détermination si le produit de la matrice C x $S^T$ correspond à la matrice de données D dans les limites de la tolérance prédéterminée.

**3.** Procédé selon la revendication 1 ou 2, dans lequel la quantité relative des solutions principales nécessaires à la création de la matrice de données C et/ou la composition déterminée des analytes des solutions principales nécessaires à la création de la matrice S est choisie sur la base de contraintes logiques et/ou de contraintes physiques et/ou de contraintes chimiques.

**4.** Procédé selon l'une quelconque des revendications 1 à 3,
dans lequel la matrice de données D est répartie en une pluralité de matrices de données Di et dans lequel chaque matrice Di est individuellement traitée dans chaque étape suivant la création de la matrice de données D, de préférence s'il est déterminé que seules des solutions principales de la matrice de données D qui sont configurées pour simuler le procédé dynamique ou l'étape de procédé spécifique ou la variante de celle-ci en dessous de la variance prédéterminée peuvent être déterminées.

5. Procédé selon l'une quelconque des revendications 1 à 4, dans lequel les données historiques comprennent au moins l'une des suivantes : valeurs de composition déterminées analytiquement du procédé dynamique ou de l'étape de procédé spécifique ou de la variante de celle-ci, informations physiques, informations de procédé, données d'au moins une variante de procédé supplémentaire correspondant à des exécutions expérimentales et/ou simulées additionnelles du procédé dans des conditions de procédé similaires ou différentes et/ou
dans lequel les données historiques sont organisées dans la matrice D selon le temps de procédé.

6. Procédé selon l'une quelconque des revendications 1 à 5, dans lequel la détermination des solutions principales nécessaires et/ou la détermination de la composition des analytes des solutions principales nécessaires et/ou la détermination de la quantité relative des solutions principales nécessaires par rapport à la matrice de données D est réalisée en utilisant un procédé chimiométrique approprié, en particulier au moins l'un des procédés chimiométriques suivants :

   un procédé de décomposition en facteurs, en particulier une analyse des composants principaux (PCA), une décomposition en valeurs singulières (SVD) ou une analyse factorielle évolutive,
   et/ou
   une analyse factorielle parallèle (PARAFAC), une résolution de courbes à variables multiples-moindres carrés alternés (MCR-ALS), une analyse factorielle évolutive.

7. Procédé selon l'une quelconque des revendications 1 à 6, comprenant en outre :
   un prétraitement de la matrice D, de préférence un filtrage, de manière davantage préférée en utilisant au moins l'un de : un filtre de Savitzky-Golay, un lisseur de Kernel, une spline de lissage, une moyenne mobile ou une moyenne mobile pondérée.

8. Procédé selon l'une quelconque des revendications 1 à 7, comprenant en outre :
   la préparation d'au moins un deuxième échantillon étant configuré pour rompre la co-linéarité entre des paramètres ou des analytes compris dans la matrice de données D en utilisant un procédé de rupture de co-linéarité.

9. Procédé selon la revendication 8, dans lequel le procédé de rupture de co-linéarité comprend au moins l'un des suivants : création d'une conception orthogonale d'expériences, ajout aléatoire des composés spécifiques dans l'au moins un échantillon, ajout programmé, mélange aléatoire d'échantillons ou toute combinaison de ceux-ci.

10. Procédé selon la revendication 8 ou 9, comprenant en outre :
    la création d'un troisième échantillon en regroupant l'échantillon avec l'au moins un deuxième échantillon.

11. Génération d'une structure d'échantillons synthétiques et système de manipulation d'échantillons pour la préparation d'au moins un échantillon synthétique simulant un procédé dynamique ou une étape de procédé spécifique ou une variante de celle-ci, le système étant adapté pour réaliser le procédé selon l'une quelconque des revendications précédentes et le système comprenant :

    une unité de calcul ; et
    une unité de mélange ;
    dans lequel l'unité de calcul comprend :

       un moyen pour la préparation de données historiques de procédé du procédé dynamique ou de l'étape de procédé spécifique ou de la variante de celle-ci à simuler et pour lesquels/laquelle l'au moins un échantillon synthétique doit être préparé ;
       un moyen de création d'une matrice de données D des données historiques de procédé ;
       un moyen de détermination d'une pluralité de solutions principales de la matrice de données D ;
       un moyen de détermination des solutions principales de la matrice de données D qui sont nécessaires pour simuler le procédé dynamique ou l'étape de procédé spécifique ou la variante de celle-ci dans les limites d'une variance prédéterminée ;
       un moyen de détermination de la composition des analytes des solutions principales nécessaires et de la quantité relative respective des solutions principales nécessaires par rapport à la matrice de données D ; et
       dans lequel l'unité de mélange est configurée pour créer l'au moins un échantillon par assemblage des solutions principales nécessaires selon la composition déterminée des analytes,

    dans lequel l'unité de calcul comprend en outre :

un moyen de création d'une matrice S comprenant la composition déterminée des analytes des solutions principales nécessaires ;

un moyen de création d'une matrice C comprenant la quantité relative des solutions principales nécessaires par rapport à la matrice de données D ;

un moyen de déterminer si le produit de la matrice C x $S^T$ correspond à la matrice de données D dans les limites d'une tolérance prédéterminée ;

dans lequel le moyen de détermination de la quantité relative respective des solutions principales nécessaires par rapport à la matrice de données D est configuré pour déterminer la quantité relative des solutions principales nécessaires comprise dans la matrice C pour au moins deux instants de temps ; l'unité de calcul comprenant en outre :

un moyen de réalisation d'une régression entre la quantité relative des solutions principales nécessaires comprise dans la matrice C et une variable de temps respective des données historiques de procédé en utilisant la quantité relative déterminée des solutions principales nécessaires pour les au moins deux instants de temps ;

un moyen d'estimation de la quantité relative des solutions principales nécessaires pour au moins un autre instant de temps entre les au moins deux instants de temps sur la base de la régression ; et

un moyen de création d'une matrice augmentée $C_{aug}$ dépendante du temps, comprenant C et les solutions principales nécessaires estimées pour l'au moins un autre instant de temps.

12. Système selon revendication 11, comprenant en outre un dispositif de mesure ayant au moins un capteur.

13. Système selon la revendication 12, dans lequel l'au moins un capteur du dispositif de mesure est configuré pour mesurer au moins un paramètre *in situ* dans le procédé dynamique, l'étape de procédé spécifique ou la variante de celle-ci et/ou dans lequel l'au moins un capteur comprend au moins l'un des suivants : une sonde de pH, une sonde de température, un capteur spectroscopique ou un instrument multivoie.

14. Système selon l'une quelconque des revendications 11 à 13, comprenant en outre au moins un dispositif de régulation comprenant au moins une structure de régulation de température et/ou au moins une structure de régulation de pH.

15. Système selon la revendication 14, dans lequel l'au moins un dispositif de régulation est configuré pour recréer les conditions respectives du procédé dynamique ou de l'étape de procédé spécifique ou de la variante de celle-ci.

16. Système selon la revendication 13 ou 14, dans lequel l'au moins un dispositif de régulation est configuré pour rompre la co-linéarité entre des paramètres ou des analytes compris dans la matrice de données D en utilisant un procédé de rupture de co-linéarité.

17. Système selon la revendication 16, dans lequel l'au moins un dispositif de régulation est configuré pour rompre la co-linéarité en utilisant au moins l'un des procédés de rupture de co-linéarité suivants : création d'une conception orthogonale d'expériences, ajout aléatoire des composés spécifiques dans l'au moins un échantillon, ajout programmé, mélange aléatoire d'échantillons ou toute combinaison de ceux-ci.

18. Système selon l'une quelconque des revendications 11 à 17 comprenant en outre un dispositif analytique configuré pour analyser au moins l'une des solutions principales et/ou l'au moins un échantillon.

19. Produit de programme d'ordinateur comprenant un ou plusieurs supports lisibles par ordinateur ayant des instructions exécutables par un ordinateur pour réaliser les étapes du procédé selon l'une quelconque des revendications 1 à 10.

Fig. 1

Fig. 1 (cont.)

Fig. 1 (cont.)

Fig. 1 (cont.)

EP 3 087 516 B1

Fig. 1 (cont.)

Fig. 1 (cont.)

Fig. 1 (cont.)

EP 3 087 516 B1

Fig. 1 (cont.)

Fig. 2

EP 3 087 516 B1

Fig. 3

Fig. 3 (cont.)

Fig. 3 (cont.)

Fig. 3 (cont.)

Fig. 3 (cont.)

Fig. 3 (cont.)

Fig. 3 (cont.)

Fig. 3 (cont.)

Fig. 4

EP 3 087 516 B1

Fig. 5

Fig. 5 (cont.)

Fig. 6

EP 3 087 516 B1

Fig. 6 (cont.)

Fig. 7

Fig. 7 (cont.)

**Prepared Historical Process Data**

**D matrix**

Containing the relative amount of each main solution to be used for preparing synthetic process samples using main solution

**C Matrix**

MCR-ALS

**S Matrix**

Containing main solution characteristics (analyte concentration, physical properties, etc.).

Information is used to assemble main solutions with the same characteristics as S matrix

% of main solution to be used in each synthetic process sample

Solution 3 – Relative amount

Solution 2 – Relative amount

Solution 1 – Relative amount

Analyte composition for each main solution

Composition of Main Solution 1

Composition of Main Solution 2

Composition of Main Solution 3

**Main Solutions**

**Synthetic Process Samples**

Main Solution 1

Main Solution 2

Main Solution 3

Fig. 8

# EP 3 087 516 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MARCELO BLANCO ; ANNA PEGUERO.** Analysis of pharmaceuticals by NIR spectroscopy without a reference method. *Trends in Analytical Chemistry,* November 2010, vol. 29 (10), 1127-1136
- **M. R. RILEY et al.** Rapid calibration of near-infrared spectroscopic measurements of mammalian cell cultivations. *Biotechnology Progress,* vol. 15 (6), 1133-1141
- **K. R. BEEBE et al.** An Introduction to Multivariate Calibration and Analysis. *Anal. Chem.,* 1987, vol. 59 (17), 1007A-1017A
- **R. N. FEUDALE et al.** Transfer of multivariate calibration models: a review. *Chemometrics and Intelligent Laboratory Systems,* 28 November 2002, vol. 64 (2), 181-192
- **P.K. HOPKE.** The evolution of chemometrics. *Analytica Chimica Acta,* 19 December 2003, vol. 500 (1-2), 365-377
- **G. M. ESCANDAR.** *Second- and third-order multivariate calibration: data, algorithms and applications*

- **DE JUAN ; ANNA ; ROMA TAULER.** Chemometrics applied to unravel multicomponent processes and mixtures. Revisiting latest trends in multivariate resolution. *Analytica Chimica Acta,* 2003, vol. 500, 195-210
- **GAMPP, H ; M MAEDER ; C J MEYER ; A D ZUBERBUHLER.** Calculation of equilibrium constants from multiwavelength spectroscopic data-IV: Model-free least squares refinement by use of evolving factor analysis. *Talanta,* 1986, vol. 33, 943-951
- **JAUMOUT ; JOAQUIM ; RAIMUNDO GARGALLO ; ANNA DE JUAN ; ROMA TAULER.** A graphical user-friendly interface for MCR-ALS: a new tool for multivariate curve resolution in MATLAB. *Chemometrics and Intelligent Laboratory Systems,* 2005, vol. 76, 101-110
- **WINDIG, W ; D A STEPHENSON.** Self-modeling mixture analysis of second-derivative near infrared spectral data using the SIMPLISMA approuch. *Analytical Chemistry,* 1992, vol. 64, 2735-2742